# FASCICULE DE BREVET EUROPEEN

(11) **EP 2 779 943 B1**
(45) Date de publication et mention de la délivrance du brevet: **11.07.2018**
(21) Numéro de dépôt: 12787735.5
(22) Date de dépôt: 16.11.2012
(51) Int. Cl.: A61F 2/24

(54) **IMPLANT DESTINÉ À ÊTRE PLACÉ DANS UN PASSAGE DE CIRCULATION DU SANG ET DISPOSITIF DE TRAITEMENT ASSOCIÉ**
IMPLANTAT ZUR POSITIONIERUNG IN EINEM BLUTFLUSSDURCHGANG UND ENTSPRECHENDE BEHANDLUNGSVORRICHTUNG
IMPLANT INTENDED FOR POSITIONING IN A BLOOD FLOW PASSAGE AND ASSOCIATED TREATMENT DEVICE

(30) Priorité: 17.11.2011 FR 1160482
(43) Date de publication de la demande: 24.09.2014
(73) Titulaire: Laboratoires Invalv, 80480 Dury (FR)
(72) Inventeur: CARMI, Doron, F-80000 Amiens (FR); PELTIER, Marcel, F-80480 Dury (FR)
(74) Mandataire: Lavoix
(86) Numéro de dépôt international: PCT/EP2012/072910
(87) Numéro de publication internationale: WO 2013/072496

(56) Documents cités:
- WO-A1-2011/057087
- WO-A2-2011/002996
- WO-A2-2011/163275
- FR-A1- 2 919 798

## Description

La présente invention est définie par les revendications. La présente invention concerne un implant destiné à être placé dans un passage de circulation du sang, l'implant étant déployable entre une configuration contractée et une configuration déployée, comprenant :
- une armature tubulaire d'axe central définissant un conduit interne de circulation du sang, l'armature tubulaire s'étendant entre une extrémité proximale et une extrémité distale ;
- une pluralité de bras distaux s'étendant perpendiculairement à l'axe central dans la configuration déployée pour s'appuyer sur une première face du tissu ;
- une pluralité de bras proximaux présentant une extrémité liée à l'armature et une extrémité libre destinée à s'appuyer sur une deuxième face du tissu pour pincer le tissu ;

Un tel implant est destiné au remplacement d'une valve cardiaque native en particulier d'une valve mitrale, par une endovalve.

Dans le cas de la valve mitrale, l'implant est destiné à être placé dans un passage sanguin auriculo-ventriculaire d'un coeur humain ou d'animal.

Lors de la systole, le passage sanguin entre l'oreillette gauche et le ventricule gauche du coeur est interrompu par la fermeture d'une valve cardiaque native présente dans un appareil mitral. Cette valve assure une circulation univoque du flux sanguin, évitant un reflux à l'issue de la contraction ventriculaire.

L'appareil mitral comprend un anneau mitral, deux feuillets valvulaires reliés à cet anneau, et un appareil sous-valvulaire comprenant des cordages et des piliers.

Les feuillets valvulaires comportent un feuillet antérieur ou « grande valve mitrale » et un feuillet postérieur ou « petite valve mitrale ».

La partie de liaison de l'anneau avec la grande valve est fibreuse alors que la partie de liaison de l'anneau avec la petite valve est musculaire. Les petites et grandes valves sont reliées à la partie ventriculaire par des cordages, eux-mêmes reliés aux piliers. En diastole, les deux feuillets s'ouvrent pour libérer le passage entre l'oreillette et le ventricule gauche.

En systole, la contraction ventriculaire engendre une brusque élévation de la pression intra-ventriculaire gauche, provoquant l'éjection du sang à travers la valve aortique. Simultanément, la contraction des piliers et la mise en tension des cordages provoquent la jonction des feuillets entre eux, de façon à isoler de manière étanche les cavités atriale et ventriculaire gauches.

Toutefois, des maladies affectent les valves et les cordages. En particulier, celles-ci peuvent souffrir d'une dégénérescence autorisant ainsi un reflux ou une régurgitation.

De plus, en cas de régurgitation mitrale sévère et chronique, le ventricule gauche sous-jacent se dilate et sa contractilité diminue, ce qui peut conduire à la nécessité d'une intervention chirurgicale cardiaque, même en l'absence de tous symptômes.

Pour pallier ces problèmes, il est connu d'implanter une endovalve entre les feuillets délimitant la valve malade. L'endovalve est par exemple constituée d'une endoprothèse tubulaire déployable et d'un obturateur souple réalisé dans un tissu d'origine animale. L'obturateur souple est fixé à demeure dans l'endo-prothèse.

De telles endovalves sont généralement implantables de manière moins invasive qu'un remplacement valvulaire chirurgical, ce qui limite les risques associés à l'implantation de la valve, notamment en termes de mortalité.

On connaît par exemple de WO 2010/121076 un implant mitral disposé dans un passage sanguin auriculo-ventriculaire en remplacement de la valve native.

Un tel implant comporte une pluralité de bras atriaux et une pluralité de bras ventriculaires disposés en regard des bras atriaux pour pincer l'anneau mitral, en prenant appui sur la face atriale des feuillets de la valve native en la plicaturant. Les bras ventriculaires sont formés par des crochets disposés à l'extrémité ventriculaire de l'armature et repliés vers l'extrémité atriale. Les bras atriaux sont formés par des boucles en forme de V s'étendant en regard des bras ventriculaires, au voisinage de ceux-ci, mais s'écartant de l'armature et des bras atriaux.

Les extrémités libres des bras ventriculaires et des bras atriaux sont disposées à l'écart l'une de l'autre et sont enfoncées respectivement dans une face atriale et dans une face ventriculaire de l'anneau mitral.

Un autre exemple d'implant est notamment décrit dans WO 2011/057087.

On notera que l'installation d'un implant mitral en remplacement de la valve native peut être réalisée en passant par la cavité atriale, ou en passant en variante par la cavité ventriculaire. Cette installation est généralement réalisée au moyen d'un outil de largage adapté. La structure de cet outil de largage peut être différente en fonction du côté (atrial ou ventriculaire) par lequel on passe pour réaliser cette installation. Un autre exemple d'implant est décrit dans WO 2011/00296. Cet implant est un appareil destiné à remplacer une valvule cardiaque malade qui peut passer d'une configuration où il est plié de manière radiale à une configuration où il est déplié de manière radiale. L'appareil comprend un élément de support extensible et une valve prothétique fixée sur celui-ci. La partie principale du corps s'étend entre une première et une seconde extrémité et comprend une surface périphérique externe, un axe périphérique s'étendant autour de la surface périphérique, et une pluralité d'éléments d'ailes espacés les uns des autres par une région extensible. Chacun des éléments d'ailes comprend une première et une seconde extrémité et une partie médiane flexible s'étendant entre les extrémités. La seconde extrémité forme un tout avec la partie principale du corps. La première extrémité est adjacente à l'axe périphérique et est pratiquement au niveau de la surface périphérique externe dans la configuration où elle est pliée de manière radiale. La première extrémité s'étend pratiquement de manière radiale en direction de la surface périphérique externe dans la configuration où elle s'étend de manière radiale. L'invention a notamment pour but de faciliter l'installation d'un implant mitral, notamment lorsque celui-ci est installé en passant par la cavité ventriculaire.

A cet effet, l'invention a notamment pour objet un implant destiné à être placé dans un passage de circulation du sang, et à être fixé sur un tissu, l'implant étant déployable entre une configuration contractée et une configuration déployée, l'implant comprenant :
- une armature tubulaire selon un axe central, définissant un conduit interne de circulation du sang, l'armature tubulaire s'étendant entre une extrémité proximale et une extrémité distale ;
- une pluralité de bras distaux s'étendant perpendiculairement à l'axe central dans la configuration déployée pour s'appuyer sur une première face du tissu ;
- une pluralité de bras proximaux présentant une extrémité liée à l'armature et une extrémité libre destinée à s'appuyer sur une deuxième face du tissu pour pincer le tissu ; caractérisé en ce que
- l'implant comprend un premier ensemble d'un seul tenant comportant une première partie de l'armature et les bras proximaux, et un deuxième ensemble d'un seul tenant comportant une deuxième partie de l'armature et les bras distaux, le premier ensemble et le deuxième ensemble étant rapportés l'un sur l'autre ;
- la première partie de l'armature présente la forme d'un manchon proximal, d'axe central, s'étendant longitudinalement entre une extrémité proximale et une extrémité distale de manchon ;
- l'extrémité liée de chaque bras proximal est liée à l'extrémité distale du manchon proximal, et l'extrémité libre de chaque bras proximal s'étend dans la direction de l'axe central au-delà de cette extrémité distale du manchon proximal.

Chaque bras proximal s'étend depuis l'extrémité distale du manchon proximal, au-delà de cette extrémité distale. Ainsi, lorsque le manchon proximal est approché des feuillets valvulaires depuis la cavité ventriculaire, les bras proximaux se trouvent en avant de ce manchon, si bien qu'ils peuvent accueillir les feuillets valvulaires sans être gênés par le manchon.

En d'autres termes, la structure d'implant telle que définie ci-dessus permet une grande facilité d'insertion des feuillets valvulaires dans l'espace de réception délimité par les bras proximaux.

L'implant selon l'invention peut comprendre l'une ou plusieurs des caractéristiques suivantes, prise(s) isolément ou suivant toute combinaison techniquement possible :
- Les bras proximaux délimitant entre eux un espace de réception du tissu, le manchon proximal s'étend intégralement en dehors de cet espace de réception.
- Les première et seconde parties de l'armature sont chacune formée par un treillis de fils entrelacés, délimitant par exemple des mailles polygonales.
- Les bras proximaux définissent entre eux un espace de réception du tissu, la seconde partie de l'armature présente la forme d'un manchon distal, d'axe central, ce manchon distal s'étendant longitudinalement : en partie à l'intérieur du manchon proximal, coaxialement à ce manchon proximal, en partie à l'intérieur de l'espace de réception de tissu, de sorte que le tissu est susceptible d'être reçu entre les bras proximaux et le manchon distal, et en partie au-delà de l'espace de réception de tissu.

De manière optionnelle, dans la configuration déployée, en l'absence de sollicitation extérieure, l'extrémité libre d'au moins un bras proximal est disposée au contact d'un bras distal et/ou de l'armature, le bras proximal comportant au moins une région intermédiaire s'étendant le long et à l'écart radialement de l'armature pour définir une loge longitudinale de réception du tissu.

En effet, il apparaît qu'un implant de l'état de la technique, par exemple tel que décrit dans WO 2010/121076, ne donne pas entière satisfaction. En particulier, la partie de liaison de l'anneau avec la grande valve étant fibreuse, les bras annulaires et ventriculaires se plantent dans un tissu peu robuste.

La fixation axiale de l'implant s'en trouve donc affaiblie, et son positionnement dans le temps peut évoluer, notamment sous l'effet de la pression du sang s'appliquant sur la valve, à l'origine d'un déplacement secondaire de l'implant.

De plus, la fixation de l'implant sur la totalité de l'anneau mitral expose en regard de sa zone antérieure un risque de gène à l'écoulement sanguin en regard de la voie d'éjection ventriculaire gauche délimitée par cette zone antérieure.

Dans le cas, évoqué ci-dessus, où l'implant selon l'invention présente au moins un bras proximal dont l'extrémité libre est disposée au contact d'un bras distal et/ou de l'armature, et où le bras proximal comporte au moins une région intermédiaire, on obtient un implant, destiné à être implanté en remplacement d'une valve native défectueuse, notamment d'une valve mitrale, qui présente une structure simple, tout en offrant une fixation robuste sur un tissu de la valve native.

En particulier, dans le cas de la valve mitrale, on offre ainsi une fixation plus large sur la surface des deux feuillets et ses deux faces atriale ou distale, et ventriculaire ou proximale, avec une position excentrée de l'implant mitral s'éloignant de la zone antérieure de l'anneau mitral et prenant appui avantageusement sur sa zone postérieure.

Avantageusement, l'implant selon l'invention présente au moins un bras proximal dont l'extrémité libre est disposée à la fois au contact d'un bras distal et au contact de l'armature. Ainsi, le bras proximal présente plusieurs points d'appui, si bien que l'implant est ancré de manière plus stable.

L'implant selon l'invention peut en outre comprendre l'une ou plusieurs des caractéristiques suivantes, prise(s) isolément ou suivant toute combinaison techniquement possible :
- La région intermédiaire de chaque bras proximal présente une forme bombée, de convexité dirigée radialement à l'écart de l'axe, la région intermédiaire comprenant au moins un tronçon proximal divergeant radialement à l'écart de l'extrémité liée et au moins un tronçon distal convergeant radialement vers l'extrémité libre.
- Au moins un bras proximal définit, au niveau de son extrémité libre, une région distale faisant saillie radialement à l'écart de l'axe central par rapport à la région intermédiaire, la région distale étant appliquée sous un bras distal, la rigidité en flexion de la région intermédiaire étant de préférence supérieure à la rigidité en flexion de la région distale.
- Chaque bras proximal comporte deux branches convergeant distalement l'une vers l'autre pour présenter sensiblement une forme de V retourné en configuration déployée.
- Chaque bras distal forme une boucle faisant saillie transversalement par rapport à l'axe central.
- Les bras distaux sont adjacents les uns aux autres pour former une collerette transversale perpendiculaire à l'axe dans la configuration déployée, la collerette étant avantageusement couverte d'une jupe en tissu propre à guider le sang à travers l'armature.
- L'étendue radiale d'un premier bras distal dans un premier secteur angulaire autour de l'axe est supérieure à l'étendue radiale d'un deuxième bras distal situé dans un deuxième secteur angulaire, avantageusement supérieure à 50% de l'étendue radiale d'un deuxième bras distal.
- Au moins un premier bras proximal présente une première région distale appliquée en regard d'un premier bras distal, un deuxième bras proximal présentant une deuxième région distale appliquée en regard d'un deuxième bras distal, l'étendue radiale de la première région distale étant supérieure à l'étendue radiale de la deuxième région distale.
- Le manchon proximal présente, lorsqu'il est séparé du manchon distal, et en l'absence de sollicitation extérieure, un diamètre inférieur à celui du manchon distal en l'absence de sollicitation extérieure.
- Chaque bras proximal est déplaçable radialement à l'écart de l'axe central depuis une position de repos au contact d'un bras distal et/ou de l'armature vers une position écartée radialement pour l'introduction du tissu dans la loge, le bras proximal étant sollicité élastiquement vers la position de repos, ou en variante étant sollicité vers la position de repos par tout moyen mécanique envisageable ;
- La longueur du ou de chaque bras proximal, prise entre son extrémité liée à l'armature et son extrémité libre le long de l'axe A-A' est supérieure à 50%, avantageusement supérieure à 70%, de la longueur de l'armature 16, prise entre l'extrémité proximale et l'extrémité distal.
- L'implant comporte un obturateur souple monté dans le conduit interne pour obturer sélectivement le passage du sang.
- L'implant est monobloc en étant venu de matière.
- La région intermédiaire définissant la loge longitudinale est située entre l'extrémité liée et le point de contact de l'extrémité libre avec l'armature et/ou le bras distal.
- La région distale est située axialement à l'opposé de la région intermédiaire par rapport au point de contact de l'extrémité libre avec l'armature et/ou le bras distal.

L'invention a également pour objet un dispositif de traitement d'un passage de circulation du sang, caractérisé en ce qu'il comporte :
- un implant tel que défini plus haut ;
- un outil de largage de l'implant, l'implant étant monté dans sa configuration contractée dans l'outil de largage.

Le dispositif de traitement selon l'invention peut comprendre l'une ou plusieurs des caractéristiques suivantes, prise(s) isolément ou suivant toute combinaison techniquement possible :
- Le dispositif comporte une tête de blocage délimitant au moins un logement de réception de l'armature, un tuteur engagé dans l'armature, une gaine interne de retenue de chaque bras distal dans une configuration axiale disposée autour de l'armature et une gaine externe de plaquage de chaque bras proximal contre la gaine externe, la gaine interne étant disposée autour de l'armature et de chaque bras distal pour maintenir le bras distal dans une configuration axiale.
- Le dispositif comporte une tête de blocage délimitant au moins un logement de réception de l'armature et de retenue de chaque bras distal dans une configuration axiale, un tuteur engagé à travers l'armature, une gaine externe de placage de chaque bras proximal contre l'armature, l'armature et les bras distaux étant sensiblement immobiles par rapport au tuteur lors du déplacement de la gaine externe, le dispositif comportant en outre une paroi intermédiaire faisant saillie à partir de la tête dans la gaine externe pour maintenir en position les bras proximaux et les séparer des bras distaux.
- Le dispositif comporte : une tête de blocage délimitant au moins un logement de réception de l'armature, un tuteur engagé dans l'armature, comprenant une extrémité distale fixée à la tête de blocage, une gaine interne de retenue de la deuxième partie de l'armature en configuration contractée, la gaine interne étant disposée autour de la deuxième partie de l'armature, une gaine externe de plaquage de chaque bras proximal contre la gaine externe, la gaine externe étant disposée autour des bras proximaux, et une gaine intermédiaire de retenue de la première partie de l'armature en configuration contractée, la gaine intermédiaire étant disposée autour de la première partie de l'armature, cette première partie étant disposée entre la gaine interne et la gaine intermédiaire.

L'invention sera mieux comprise à la lecture de la description qui va suivre, donnée uniquement à titre d'exemple, et faite en se référant aux dessins annexés, sur lesquels :
- la Figure 1 est une vue de côté d'un premier implant selon l'invention, dans une configuration déployée ;
- la Figure 2 est une vue schématique, prise en coupe partielle suivant un plan axial médian de l'implant de la Figure 1 ;
- la Figure 3 est une vue en développée d'une partie inférieure de l'implant de la Figure 1 dans la configuration déployée de l'implant ;
- la Figure 4 est une vue analogue à la Figure 3 d'une partie supérieure de l'implant dans la configuration contractée de l'implant ;
- la Figure 5 est une vue de côté illustrant le profil latéral des bras proximaux latéraux ;
- la Figure 6 est une vue de dessus de l'implant représenté sur la Figure 1, illustrant la collerette distale ;
- la Figure 7 est une vue d'un dispositif de traitement, dans lequel l'implant de la Figure 1 est chargé dans une configuration contractée ;
- les Figures 8 à 11 illustrent différentes phases de largage de l'implant de la Figure 1 dans un passage auriculo-ventriculaire, et
- la Figure 12 est une vue analogue à la Figure 7 d'une variante de dispositif de traitement selon l'invention ;
- les Figures 13 à 15 sont des vues schématiques analogues aux Figures 8 à 11 illustrant différentes phases de largage à l'aide du dispositif de la Figure 12 ;
- la Figure 16 représente schématiquement, en coupe axiale, un dispositif de traitement selon une autre variante de réalisation, dans lequel l'implant est chargé dans une configuration contractée ;
- les Figures 17 à 22 sont des vues schématiques analogues à la Figure 16, représentant le dispositif de traitement de la Figure 16 dans différentes phases de largage ;
- la Figure 23 est une vue schématique de profil d'un implant selon une variante de réalisation de celui des figures 3 et 4, disposé dans un passage de circulation du sang ;
- les Figures 24 à 26 sont des vues schématiques analogues aux figures 16 à 22, représentant un dispositif de traitement comprenant l'implant de la figure 23, dans différentes phases de largage de cet implant ;
- les Figures 27 et 28 sont des vues schématiques similaires à la Figures 23, représentant des implants selon des variantes de réalisation respectives, disposés dans un passage de circulation du sang ;
- les Figures 29 et 30 sont des vues schématiques de profil d'une première partie d'un implant selon des variantes de réalisation respectives ;
- les Figures 31 et 32 sont des vues analogues aux Figures 3 et 4, représentant respectivement une première et une seconde partie d'un implant selon une autre variante de réalisation.

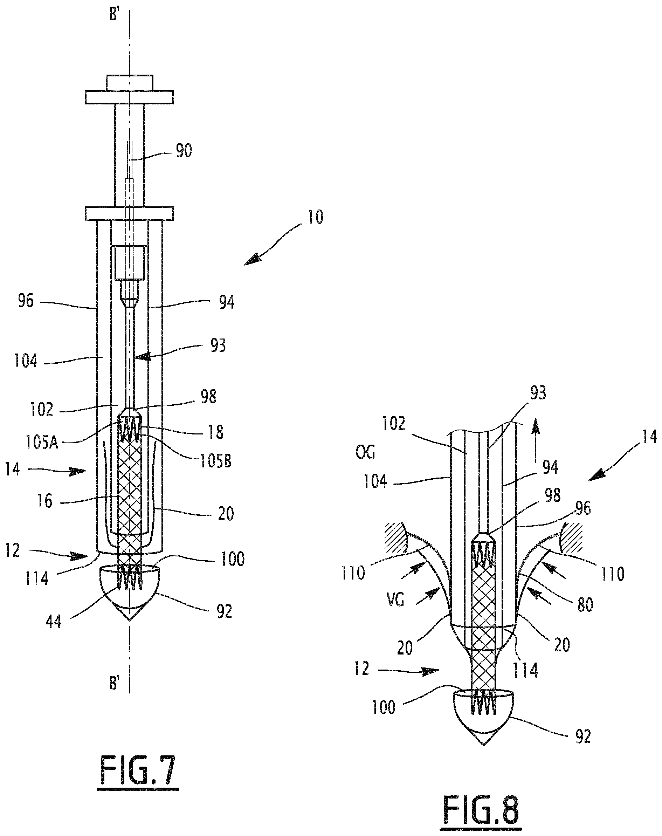

Un premier dispositif de traitement 10 selon l'invention est illustré par la Figure 7.

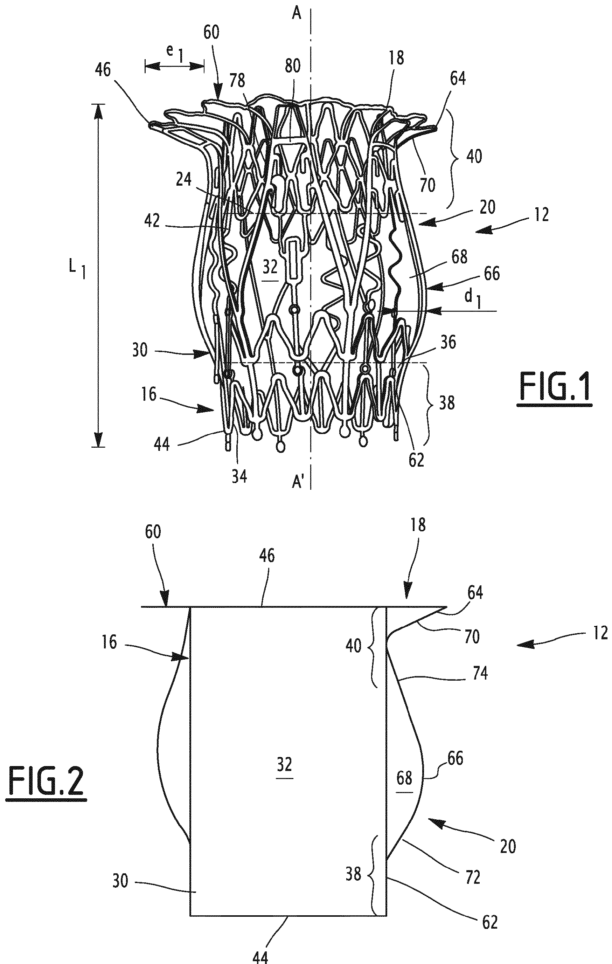

Le dispositif 10 comporte un implant 12, visible en détail sur les Figures 1 à 6, et un outil 14 de largage de l'implant 12 destiné à positionner et à déployer l'implant 12 dans un passage de circulation du sang, par exemple dans un passage situé dans le coeur d'un patient.

L'implant 12 est avantageusement une endovalve, notamment une valve cardiaque destinée à remplacer une valve native défectueuse. L'endovalve est avantageusement une valve auriculo-ventriculaire, destinée à remplacer une valve mitrale native située entre l'oreillette gauche et le ventricule gauche, de façon à permettre une circulation univoque du flux sanguin entre la cavité atriale et le volume ventriculaire gauche.

En variante, l'endovalve est une valve atrio-ventriculaire destinée à remplacer la valve cardiaque en position tricuspide.

Comme illustré par la Figure 1 et 2, l'implant 12 comporte une armature tubulaire 16, une pluralité de bras distaux 18 destinés à former un appui distal sur une face distale d'un feuillet de valve, et une pluralité de bras proximaux 20 destinés à recevoir une face proximale des feuillets de la valve native pour s'accrocher sur cette valve.

Dans le cas où la valve native est la valve mitrale, la face distale constitue la face atriale et la face proximale constitue la face ventriculaire.

L'implant 12 comporte en outre avantageusement un obturateur 24 à base de tissu, notamment de tissu synthétique ou naturel, tel que bovin, équin et/ou de péricarde porcin. L'obturateur 24 est représenté schématiquement en pointillés sur la Figure 2. Il est destiné à assurer la circulation univoque du sang à travers l'implant 12.

L'implant 12 s'étend généralement suivant un axe central A-A'. Il est déployable entre une configuration contractée, qu'il occupe lorsqu'il est disposé dans l'outil de largage 14, et une configuration déployée, qu'il occupe lorsqu'il est situé hors de l'outil de largage 14.

Dans une variante avantageuse, l'implant 12 est auto-expansible, c'est-à-dire que sa configuration déployée constitue sa position de repos, l'implant 12 dans sa configuration contractée étant sollicité vers sa configuration déployée.

L'armature 16, les bras 18, et les bras 20 sont formés par exemple d'un acier inoxydable ayant des propriétés élastiques. En variante, ces éléments sont formés à base de métal à mémoire de forme tel que du Nitinol ou d'une fibre souple polymère.

L'armature tubulaire 16 est formée par un bâti tubulaire 30 définissant intérieurement un conduit de circulation du sang 32.

Le bâti 30 est avantageusement réalisé à base d'une pluralité d'éléments filiformes 34 ou monobloc formant une paroi périphérique d'axe A-A' et délimitant entre eux des ouvertures de passage 36.

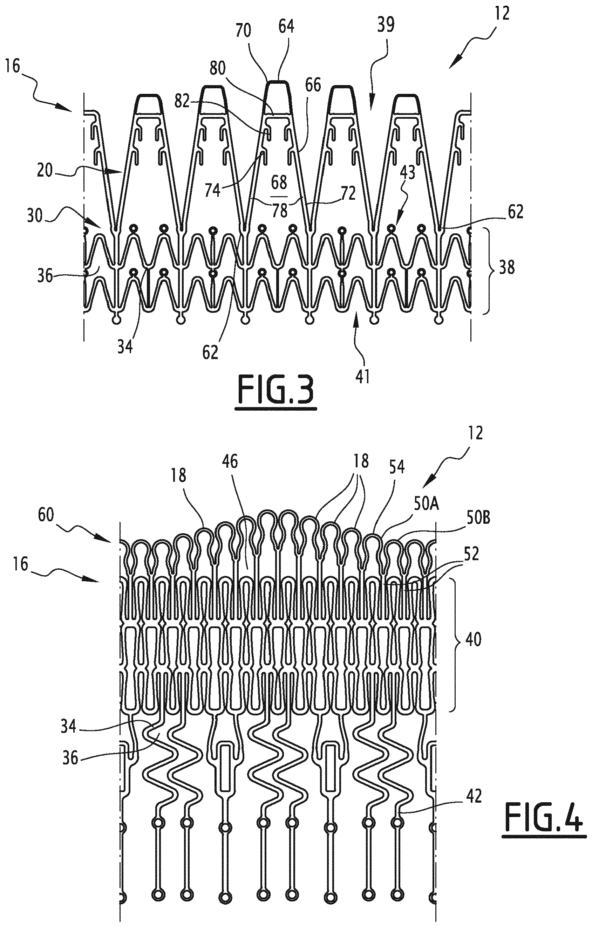

Dans l'exemple représenté sur les Figures 3 et 4, le bâti 30 comporte un manchon proximal 38 formé d'éléments filiformes 34 ondulés, un manchon distal 40 formé d'éléments filiformes 34 ondulés et une pluralité de membrures 42 longitudinales raccordant le manchon proximal 38 au manchon distal 40.

Chaque manchon 38, 40 comprend une pluralité de rangées d'éléments filiformes 34 ondulés raccordés entre eux par des pattes longitudinales.

En variante, l'armature 16 est formée par un treillis de fils entrelacés délimitant par exemple des mailles polygonales.

Dans cet exemple, le manchon proximal 38 et les bras proximaux 20 forment un premier ensemble d'un seul tenant, visible sur la Figure 3. Cet ensemble est rapporté sur un deuxième ensemble d'un seul tenant visible sur la Figure 4, et formé par les membrures 42, le manchon distal 40 et les bras distaux 18.

Le premier ensemble et le deuxième ensemble sont rapportés l'un sur l'autre, puis de manière optionnelle solidarisés par des sutures ou autre moyen de liaison.

Avantageusement, le manchon proximal 38 présente, lorsqu'il est séparé du manchon distal 40, et en l'absence de sollicitation extérieure, un diamètre inférieur à celui du manchon distal 40 en l'absence de sollicitation extérieure. Ainsi, lorsque le manchon distal 40 est déployé à l'intérieur du manchon proximal 38, il exerce un effort radial sur une surface interne de ce manchon proximal 38, cet effort radial étant suffisant pour assurer la liaison entre le manchon proximal 38 et le manchon distal 40.

A cet effet, on notera que le manchon distal 40 est par exemple formé d'un acier inoxydable présentant des propriétés élastiques. Par exemple, le manchon distal 40 est déployé par l'inflation d'un ballon gonflable.

En variante, le manchon distal 40 pourrait être formé d'un métal à mémoire de forme, tel que du nitinol (Nickel/Titane).

Par ailleurs, le manchon distal 40 présente une longueur, dans la direction de l'axe A-A', supérieure à la longueur du manchon proximal 38. Ainsi, le manchon proximal 38 peut être disposé selon différentes positions sur le manchon distal 40, notamment en fonction de la configuration prédéterminée du passage de circulation de sang destiné à recevoir l'implant 12.

Comme cela est notamment représenté sur la Figure 3, le manchon proximal 38, s'étend longitudinalement entre une extrémité proximale 41 et une extrémité distale 43 de manchon. Chaque bras proximal 20 s'étend entre une extrémité 62 liée à l'extrémité distale 43 du manchon proximal 38, et une extrémité libre 64. Ainsi, chaque bras proximal 20 s'étend dans la direction de l'axe central A-A' au-delà de cette extrémité distale 43 du manchon proximal 38.

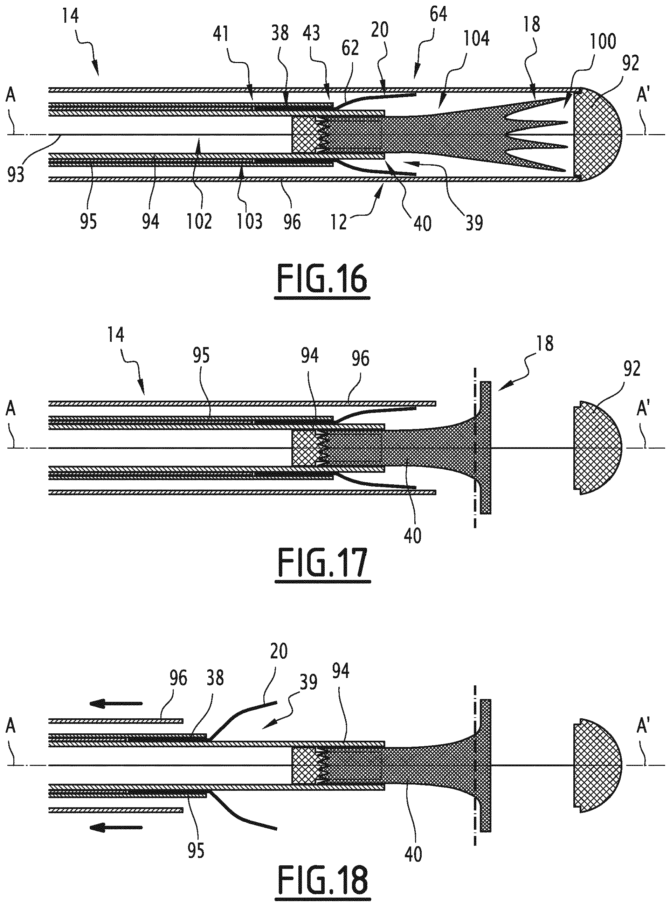

Une telle configuration permet notamment de faciliter l'insertion des feuillets de valve dans un espace de réception 39 de ces feuillets de valve délimité entre les bras proximaux 20, comme cela sera décrit ultérieurement en référence aux figures 16 à 22 et 24 à 26.

En particulier, on notera que le manchon proximal 38 s'étend intégralement en dehors de cet espace de réception 39, si bien qu'il ne gêne pas l'insertion des feuillets de valve dans cet espace de réception 39.

La section transversale de l'armature tubulaire 16 et celle du conduit 32 est avantageusement sensiblement constante.

Le conduit 32 s'étend à travers l'armature 16 le long de l'axe A-A'. Il débouche axialement à une extrémité proximale 44 de l'armature 16 et à une extrémité distale 46 de l'armature 16. On notera que l'extrémité proximale 44 de l'armature 16 peut être formée par l'extrémité proximale 41 du manchon proximal 38, ou par l'extrémité proximale du manchon distal 40, en fonction de la disposition relative de ces manchons proximal 38 et distal 40. Par ailleurs, l'extrémité distale 46 de l'armature 16 est généralement formée par l'extrémité distale du manchon distal 40.

Dans la configuration contractée, la section du conduit 32 est minimale. La longueur L1 de l'armature tubulaire 16, prise le long de l'axe A-A' entre ses extrémités 44, 46, est alors maximale. Au contraire, dans la configuration déployée, la longueur L1 de l'armature tubulaire 16 est minimale et la section transversale du conduit 32 est maximale.

Dans la configuration déployée, la longueur L1 de l'armature 16, prise entre ses extrémités 44, 46 le long de l'axe A-A', est supérieure à 10 mm et est notamment comprise entre 5 mm et 45 mm pour s'adapter à une variété de conformations anatomiques.

Comme illustré par les Figures 1, 2 et 4, les bras distaux 18 s'étendent radialement à partir de l'armature tubulaire 16 au voisinage de l'extrémité distale 46, par exemple à une distance inférieure à 10% de la longueur L1 de l'armature par rapport à l'extrémité distale 46.

Les bras distaux 18 sont répartis à la périphérie de l'armature 16. Le nombre de bras distaux est supérieur à 2, et est notamment compris entre 5 et 20.

Dans l'exemple représenté sur la Figure 4, les bras 18 s'étendent continûment sur toute la périphérie de l'armature tubulaire 16 autour de l'axe A-A' en étant adjacents les uns aux autres.

Chaque bras distal 18 est formé par une boucle 50A, 50B présentant deux tronçons 52 intérieurs communs avec un bras distal 18 adjacent, et un tronçon extérieur propre 54 replié en boucle.

Comme illustré par les Figures 1 et 4, les bras distaux 18 sont mobiles entre une position axiale, représentée sur la Figure 4, lorsque l'implant 12 occupe sa configuration contractée, et une position transversale, représentée sur la Figure 1, lorsque l'implant 12 occupe sa configuration déployée.

Dans la position transversale, au repos, en l'absence de sollicitation extérieure, chaque bras distal 18 s'étend perpendiculairement à l'axe A-A'.

Dans cette position, l'étendue e1 de chaque bras distal 18, prise entre l'armature 16 et l'extrémité libre du bras 18, perpendiculairement à l'axe A-A', est inférieure à 50% de la longueur L1.

En variante, l'étendue e1 des bras distaux 18 peut être encore plus grande, notamment supérieure à 50% du diamètre de l'armature tubulaire 16. Une telle étendue permet de déborder au-delà de l'anneau mitral et de prendre appui sur les parois de l'oreillette gauche.

Avantageusement, chacun des bras distaux 18 peut comporter une région distale, plus souple qu'une région proximale formant une patte légèrement concave. La concavité est alors orientée vers l'oreillette gauche et la patte est appliquée au-delà de l'anneau mitral sur les parois de l'oreillette gauche. Dans cette configuration, chaque bras distal 18 présente avantageusement une étendue radiale supérieure à 80% de la longueur L1 de l'armature 16.

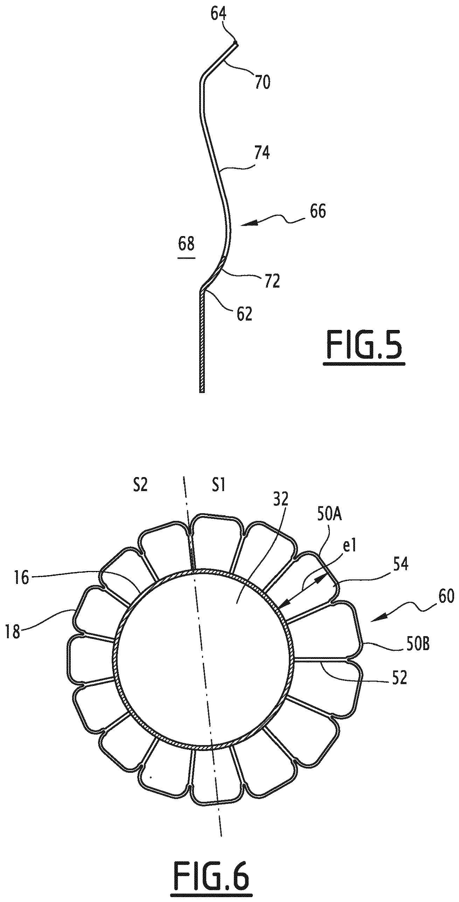

Comme illustré par la Figure 6, l'étendue radiale e1 des bras 18 dans un premier secteur angulaire S1 est par ailleurs supérieure à l'étendue radiale e1 des bras 18 situés dans un deuxième secteur angulaire S2. Ceci permet de s'adapter à la conformation anatomique du siège de la valve native, notamment lorsque la valve native est la valve mitrale.

En configuration déployée, les bras distaux 18 forment ainsi une collerette 60 annulaire continue destinée à prendre appui sur le versant distal de la valve native, sur le versant atrial, sur l'anneau et sur la paroi de l'oreillette lorsque la valve native est la valve mitrale.

Avantageusement, une jupe en tissu, notamment en Dacron ou en un autre tissu synthétique ou biologique recouvre la collerette 60. Cette jupe assure ainsi une étanchéité entre l'oreillette gauche et le ventricule gauche au pourtour de l'armature tubulaire 16. La jupe est avantageusement cousue sur les bras distaux 18 et peut éventuellement déborder radialement au-delà de la collerette 60 pour s'appuyer sur la face atriale de l'anneau mitral et sur les faces internes des parois de l'oreillette gauche.

La jupe peut également couvrir la surface interne de l'armature 16 jusqu'à l'obturateur 24 pour garantir le passage du sang à travers l'obturateur 24 sans risque de fuite à travers la paroi de l'armature 16.

Dans l'exemple représenté sur les Figures, l'implant 12 comporte une pluralité de bras proximaux 20 espacés angulairement les uns des autres autour de l'axe A-A'.

Le nombre de bras proximaux 20 est inférieur ou égal au nombre de bras distaux 18. Dans cet exemple, le nombre de bras proximaux 10 est supérieur à 2, et est notamment compris entre 4 et 10.

Chaque bras proximal 20 s'étend axialement entre une extrémité 62 liée à l'armature 16, située au voisinage de l'extrémité proximale 44 et une extrémité libre 64 destinée à venir en appui contre un bras distal 16 ou contre l'armature 16 lorsque l'implant 12 occupe sa configuration déployée.

La distance séparant le long de l'axe A-A' l'extrémité proximale 44 de l'armature 16 de l'extrémité liée 62 est inférieure à 20% de la longueur L1 de l'armature 16.

Chaque bras proximal 20 présente ainsi une longueur, prise parallèlement à l'axe A-A' le long de l'armature 16 supérieure à 50%, avantageusement supérieure à 70% de la longueur L1 de l'armature tubulaire 16, prise entre l'extrémité proximale et l'extrémité distale.

Comme illustré par la Figure 5, chaque bras proximal 20 comporte, entre son extrémité liée 62 et son extrémité libre 64, au moins une région intermédiaire 66 s'étendant le long et à l'écart radialement de l'armature 16 pour définir une loge 68 longitudinale de réception d'un feuillet de valves.

Avantageusement, au moins un bras proximal 20 comporte en outre une région distale 70 formant une patte extérieure appliquée sous un bras distal 18, ou sur l'armature 16.

Comme illustré par les Figures 1 et 5, au moins un bras 20 présente ainsi un profil en forme de S avec une région intermédiaire 66 bombée extérieurement par rapport à l'axe A-A' et une région distale 70 faisant saillie radialement à l'écart de l'axe A-A' par rapport à la région intermédiaire 66.

Comme illustré par les Figures 1 et 5, la région intermédiaire 66 comprend un tronçon proximal 72 divergent radialement à l'écart de l'axe A-A' s'étendant à partir de l'extrémité liée 62, et un tronçon distal 74 convergent vers l'axe A-A' en se déplaçant le long de l'axe A-A' depuis l'extrémité liée 62 vers l'extrémité libre 64.

La distance maximale d1 séparant radialement la région intermédiaire 66 de l'enveloppe extérieure de l'armature tubulaire 16 est supérieure à 10 % du diamètre de l'armature tubulaire. Cette distance est avantageusement inférieure à 40 % du diamètre de l'armature tubulaire 16.

La région distale 70 forme une patte qui diverge radialement vers l'extérieur à partir du tronçon convergent 74.

Dans la configuration déployée de l'implant 12, chaque région distale 70 fait saillie radialement sous un bras distal 18.

La région distale 70 présente une souplesse en flexion supérieure à celle de la région intermédiaire 66. L'élément filiforme formant la région distale 70 présente par exemple une épaisseur maximale inférieure à l'épaisseur maximale de l'élément filiforme formant la région intermédiaire 66.

L'étendue radiale maximale de la région distale 70, prise entre l'extrémité du tronçon distal 74 et l'extrémité libre 64 de la région distale 70 est supérieure à l'étendue radiale de la région intermédiaire 66.

En outre, les bras proximaux 20 situés dans le premier secteur angulaire S1 comportent une région distale 70 d'étendue radiale supérieure à l'étendue radiale des régions distales 70 des bras proximaux 20 situés dans le deuxième secteur angulaire S2, en correspondance avec la longueur des bras distaux 18.

Selon l'invention, dans la configuration déployée de l'implant 12, chaque bras proximal 20 est déformable radialement, entre une position de repos radialement contractée et une position écartée radialement de la position de repos, en vue de l'insertion des feuillets de la valve native dans la loge 68.

Dans la position de repos, chaque bras proximal 20 est appliqué contre l'armature 16 ou/et contre un bras distal 18 en l'absence de sollicitation extérieure.

Dans la position déployée, chaque bras 20 est rappelé élastiquement vers sa position de repos, la position de repos constituant sa position stable.

La longueur du tronçon convergent 74, prise le long de l'axe A-A' est supérieure à la longueur du tronçon divergent 72. La longueur de la région distale 70 est inférieure à la longueur du tronçon convergent 74 et à la longueur du tronçon divergent 72.

Dans l'exemple représenté sur les Figures 1 et 3, chaque bras 20 est formé par des branches linéaires 78 formant un V retourné convergent distalement. Un tronçon transversal d'extrémité 80 raccorde les branches 78 entre elles. La région distale 70, lorsqu'elle est présente, fait saillie à partir du tronçon d'extrémité 80.

Les branches 78 de deux bras proximaux 20 adjacents sont en contact l'une avec l'autre au niveau de leurs extrémités liées 62.

L'angle maximal formé par les branches 78 est par exemple inférieur à 45°, et est notamment inférieur à 30°.

Dans l'exemple représenté sur les Figures, chaque bras proximal 20 est en outre muni d'éléments d'ancrage 82 dans un feuillet de valve formés chacun par une pointe faisant saillie vers l'extrémité proximale 44.

De manière classique, l'obturateur 24 est formé par des volets souples (non représentées) fixées sur l'armature 16 dans le conduit 32. Chaque volet est par exemple formé d'un film de polymère ou d'une couche de film organique tel que du péricarde d'un animal, notamment du péricarde de veau. Les volets sont déformables dans une position d'obturation, dans laquelle le flux sanguin depuis l'extrémité proximale vers l'extrémité distale est empêché, et une position de passage du flux sanguin, dans laquelle les volets s'écartent les unes des autres.

Les volets sont fixés partiellement sur l'armature 16 suivant une ligne de suture. Le bord distal des volets est situé à distance axialement du bord distal de l'armature. La distance séparant axialement le bord distal de l'armature 16 de l'extrémité distale des volets est par exemple supérieure à 10%, notamment sensiblement égale à 20% de la longueur L1.

Comme illustré par la Figure 7, l'outil de largage 14 du premier dispositif 10 comporte une tige interne 90 munie d'une tête 92 de maintien d'une extrémité de l'implant 12, un tuteur 93 monté coulissant coaxialement sur la tige 90, une gaine intérieure 94 montée coulissante par rapport au tuteur 93 et une gaine extérieure 96 montée coulissante autour de la gaine intérieure 94, le long d'un axe B-B' d'introduction.

Le tuteur 93 et les gaines 94, 96 sont déplaçables à coulissement le long de l'axe B-B', indépendamment l'une de l'autre, et par rapport à la tige 90.

Des organes de verrouillage (non représentés) sont prévus entre la tige et le tuteur 93, entre le tuteur 93 et les gaines 94, 96 pour éviter le coulissement spontané du tuteur 93, des gaines externes 96 et internes 94. Ceci permet de procéder par étapes successives aux retraits de la gaine externe 96, de la gaine interne 94, et à la libération de l'extrémité proximale 44.Le tuteur interne 93 comporte une butée 98 de blocage axial s'étendant en regard de la tête 92. La butée 98 est destinée à bloquer l'extrémité distale 46 de l'implant 12.

La tête 92 délimite un logement 100 de réception de l'extrémité proximale 44 de l'implant 12, dans lequel l'extrémité proximale 44 est maintenue comprimée radialement. Cette extrémité 44 est par ailleurs fixée axialement par rapport au tuteur 93.

Le tuteur 93 et la gaine interne 94 délimitent un espace annulaire interne 102 destiné à recevoir l'armature 16 et les bras distaux 18. La gaine externe 96 délimite un espace annulaire externe 104 destiné à recevoir les bras proximaux 20.

Le tuteur 93 est muni d'au moins une butée 105A, 105B d'indexation angulaire de l'implant 12 pour fixer angulairement l'implant 12 par rapport au tuteur 90 dans la gaine interne 94 autour de l'axe du tuteur 90. La ou chaque butée d'indexation 105A, 105B est avantageusement radio opaque pour repérer la disposition des bras atriaux 20 de tailles inégales.

Ainsi, dans l'exemple représenté sur la Figure 7, le tuteur 93 comporte deux butées 105A, 105B diamétralement opposés décalées axialement le long de l'axe B-B'.

La butée 105A située la plus éloignée de la tête 92 est destinée à recevoir les bras proximaux 18 de plus grande étendue e1 alors que la butée 105B située la plus proche de la tête est destinée à recevoir les bras proximaux 18 de plus faible étendue e1. L'opérateur du dispositif peut ainsi orienter l'implant lors de sa mise en place dans le passage de circulation du sang.

Il peut ainsi placer les bras distaux 18 les plus longs dans la zone postérieure de l'anneau mitral lorsque l'implant 12 est destiné à remplacer la valve mitrale.

Dans la configuration contractée, lorsque l'implant 12 est reçu dans l'outil de largage 14, la longueur de l'armature 16 est maximale, et son diamètre extérieur est minimal. Les bras distaux 18 occupent alors leur position axiale, sensiblement parallèle à l'axe A-A'.

Les bras proximaux 20 sont plaqués axialement le long de l'axe A-A'. Le diamètre externe de l'implant 12 est alors minimal.

Dans cette configuration, l'armature 16 est reçue dans l'espace annulaire interne 102 séparant le tuteur 93 de la gaine interne 94. Elle est maintenue en position par la gaine 94. Les bras distaux 18 sont placés au contact de la butée 98, ou au voisinage de celle-ci en position axiale.

Les bras proximaux 20 sont plaqués contre la gaine interne 94 dans l'espace annulaire extérieur 104. L'extrémité proximale 44 de l'armature 16 fait saillie dans le logement 100 de la tête 92 et est fixée axialement par rapport au tuteur 93. La tête 92 et la tige 50 sont bloquées axialement par rapport au tuteur 93, la tête 92 étant proche de la butée 98.

Lorsque l'implant 12 doit être positionné, notamment en remplacement d'une valve native, il est introduit entre les feuillets 110 de la valve native autour du siège 112 de la valve, comme représenté sur la Figure 8. Cette introduction peut être effectuée, à l'aide du dispositif de la Figure 7 par voie transatriale, en passant à travers l'oreillette gauche.

La tête 92 et la partie aval de l'outil 14 est introduite dans le ventricule gauche, au-delà des feuillets 110, pour que toute la longueur des bras proximaux 20 soit disposée dans le ventricule gauche au-delà des feuillets 110 de la valve.

Puis, la gaine externe 96 est rétractée axialement à l'écart de la tête 92 par rapport à la gaine interne 94 et par rapport au tuteur 93 pour découvrir progressivement les bras proximaux 20.

Lorsque les bras 20 sont totalement découverts, la gaine externe 96 est alors redéplacée vers la tête 92 pour que son bord libre 114 s'intercale entre les bras 20 et l'armature 16, afin d'écarter les bras 20 de leur position contractée de repos.

Les bras 20 passent alors dans la position déployée représentée sur la Figure 8.

Dans cette position, les feuillets 110 de la valve native s'intercalent entre la gaine externe 96, l'armature tubulaire 16 et les bras proximaux 20 pour être reçus dans les loges 68.

Ensuite, l'outil 14 est déplacé vers l'oreillette gauche pour plaquer les bras ventriculaires 20 contre la face ventriculaire des feuillets 110 et ancrer les pointes 82 dans les feuillets 110.

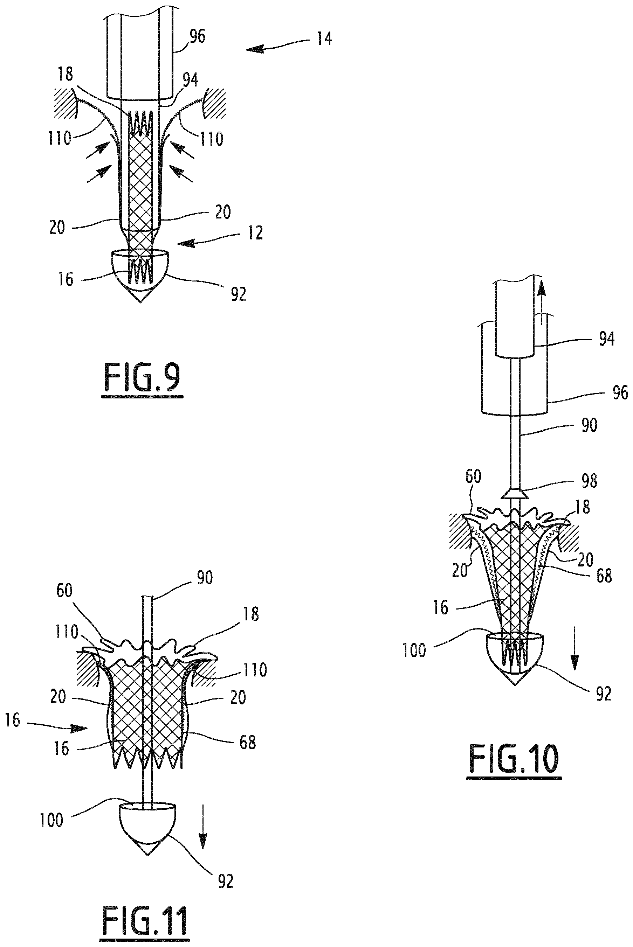

Ceci étant fait, la gaine 96 est à nouveau retirée, comme visible sur la Figure 9. Sous l'effet de la force de rappel élastique de chaque bras proximal 20 vers sa position de repos, les bras proximaux 20 se rétractent vers l'armature 16 et pincent ainsi les feuillets 110 de manière robuste.

La longueur des bras proximaux 20 étant supérieure à 50% de la longueur de l'armature 16, les feuillets 110 sont maintenus sur une grande longueur contre l'armature 16. Ils adoptent une conformation sensiblement axiale, parallèle à l'axe de l'armature 16.

Puis, la gaine interne 94 est rétractée par rapport au tuteur 93 pour découvrir progressivement l'armature 16, puis les bras distaux 18.

Comme illustré par la Figure 10, ceci provoque le déploiement radial partiel de l'armature 16 et le déploiement radial des bras distaux 18 vers leur position transversale pour prendre appui sur la face atriale des feuillets 110.

Ceci étant fait, la fixation axiale entre le tuteur 93 et l'extrémité proximale 44 est libérée par déplacement axial de la tige 90 et de la tête 92 à l'écart de la butée 98. L'armature 16 se déploie totalement. Le tuteur 93 et la tête 92 et la tige 91 sont alors retirés hors du patient à travers le conduit interne 32.

Dans la configuration déployée représentée sur la Figure 11, les bras distaux 18 forment ainsi une collerette annulaire 60 transversale prenant un appui robuste sur la face atriale des feuillets 110, et sur la face atriale de l'anneau mitral en zone postérieure et éventuellement sur la paroi auriculaire.

Les bras proximaux 20 sont alors sollicités vers leur position de repos. La région intermédiaire 66 bombée s'étendant sensiblement à l'écart et en regard de l'armature 16, sur une longueur substantielle de l'armature 16, est parfaitement conformée pour accueillir le feuillet 110 de valve native et le plaquer contre l'armature 16. La force de sollicitation élastique de chaque bras proximal 20 vers sa position de repos applique un pincement robuste sur chaque feuillet 110, assurant une réception efficace et robuste de l'implant 10 dans la valve native. Ceci est le cas, même si la valve native présente une structure fibreuse et un tissu peu robuste.

La conformation axiale particulière des bras proximaux 20, définissant chacun une loge 68 de réception du feuillet de valve sur une longueur significative par rapport à la longueur de l'implant 12, et l'obturation distale de la loge 68 par une collerette distale annulaire 60 transversale par rapport à l'axe A-A' contribuent à assurer une fixation robuste des feuillets 110. L'implant 10 est en outre simple à implanter et sûr.

Avantageusement, la fixation robuste de l'implant 12 est obtenue en l'absence de force radiale sur la totalité de l'anneau mitral, ce qui évite une dilatation de l'anneau mitral et donc l'aggravation de la régurgitation compte tenu de l'étendue radiale dissymétrique des bras proximaux distaux 18, l'armature tubulaire 16 s'implante de manière excentrée pour avantageusement prendre appui sur l'anneau mitral en zone postérieure, ce qui l'éloigne de la zone antérieure et évite le risque d'obstruction de la voie d'éjection ventriculaire gauche.

Chacun des deux feuillets natifs 110 est pincé avantageusement sur ces deux faces atriales et ventriculaires par respectivement les bras distaux 18 et les bras proximaux 20. De plus, l'anneau mitral est avantageusement pincé en zone postérieure par les bras distaux 18 et les bras proximaux 20. Ceci permet de décaler transversalement l'axe de l'armature 16 par rapport au centre de l'anneau mitral et d'éviter que la partie proximale de l'armature ne perturbe l'écoulement sanguin de la voie d'éjection ventriculaire gauche.

L'implant 12 étant fixé essentiellement sur les feuillets natifs, des bras distaux 18 et des bras proximaux 20, il n'est pas nécessaire de disposer d'un appui total sur l'anneau mitral, ce qui permet de s'affranchir de la taille de cet anneau pour l'implantation de l'implant 12. L'implant 12 forme alors un réducteur qui présente un diamètre d'armature avantageusement inférieur au diamètre de l'anneau mitral dans lequel il est importé. Ceci équivaut à la formation d'un entonnoir mitral, ou la ligne de section distale correspond au pourtour de l'anneau mitral natif et la ligne de section proximale correspond au diamètre de l'armature tubulaire.

Le sang est alors guidé à travers l'obturateur 24 par l'intermédiaire de la jupe.

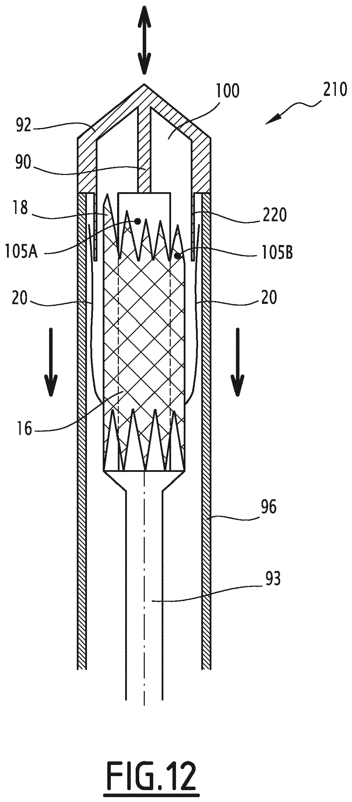

Un deuxième dispositif de traitement 210 recevant un implant 12 est illustré par la Figure 12. A la différence du dispositif 10 représenté sur la Figure 7, ce dispositif 210 est destiné à être implanté à travers le ventricule gauche, puis à travers l'oreillette gauche par la pointe du coeur, dans sa forme déployée.

Dans ce dispositif, les bras distaux 18 sont reçus dans le logement 100 de la tête 92 et sont fixés axialement par rapport au tuteur 93. Le dispositif est dépourvu de gaine intermédiaire 94. Une paroi intermédiaire 220 s'étend à partir de la tête 92 pour maintenir en position les bras proximaux 20 et les séparer des bras distaux 18.

Le largage de l'implant 12 contenu dans le dispositif 210 s'effectue depuis le ventricule gauche vers l'oreillette gauche à travers la valve native. Puis, la gaine externe 96 est rétractée pour découvrir les bras proximaux 20 comme décrit précédemment. Si l'implant 12 n'est pas correctement positionné, la gaine externe 96 peut être redéplacée vers la tête 98, afin de couvrir à nouveau les bras proximaux 20 et replacer ces bras 20 dans la gaine externe 96.

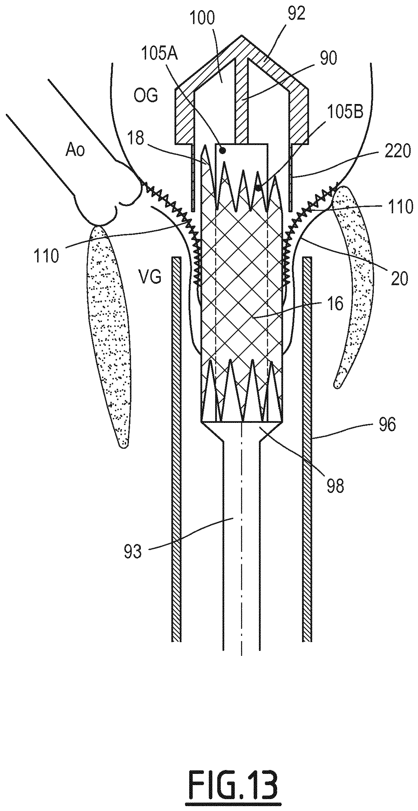

Le positionnement angulaire de l'implant 12 est obtenu grâce aux butées radio-opaques 105A, 105B visibles sur la Figure 13.

La tête 98 est ensuite rétractée par rapport au tuteur 93 pour que la paroi intermédiaire 220 coopère avec les bras proximaux 20 et les écarte de leur position de repos. Comme illustré par la Figure 13, les bras proximaux 20 sont alors déplacés pour insérer les feuillets 110 dans les loges 68.

Comme précisé précédemment, l'axe de l'armature 16 est décalé par rapport au centre de la valve mitrale, grâce à la différence de taille entre les bras distaux 18.

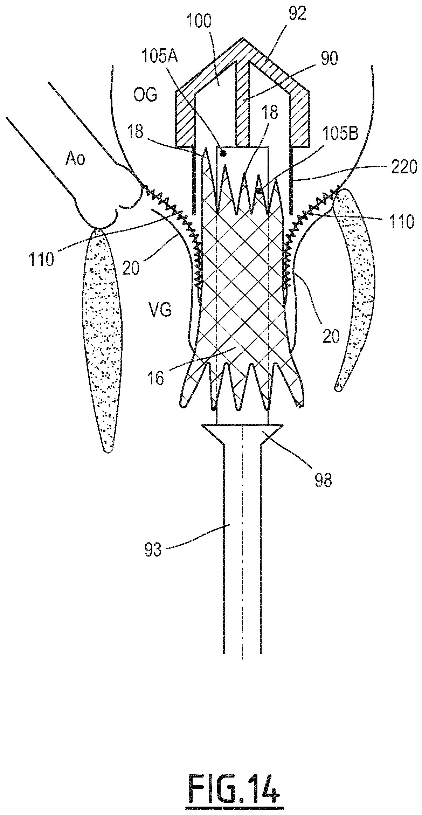

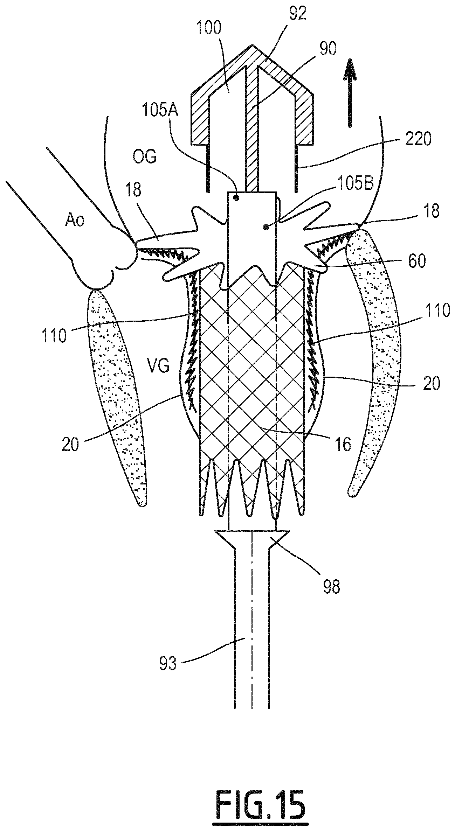

Une fois les bras proximaux 20 logés contre la face ventriculaire des feuillets 110 de la valve (Figure 14), la tête 92 est à nouveau déplacée conjointement avec la tige 90 par rapport au tuteur 93 vers l'oreillette pour permettre le déploiement des bras distaux 18 comme illustré par la Figure 15.

Un autre dispositif de traitement, recevant un implant 12, est représenté sur la figure 16. Sur cette figure, et les suivantes, les éléments analogues à ceux des figures précédentes sont désignés par des références identiques.

Comme indiqué précédemment, l'implant 12 comporte un premier ensemble d'un seul tenant comportant le manchon proximal 38 de l'armature et les bras proximaux 20, et un deuxième ensemble d'un seul tenant comportant le manchon distal 40 de l'armature et les bras distaux 18.

Le manchon proximal 38 s'étend, le long de l'axe central A-A', entre son extrémité proximale 41 et son extrémité distale 43. L'extrémité liée 62 de chaque bras proximal 20 est liée à l'extrémité distale 43 du manchon proximal 38, et l'extrémité libre 64 de chaque bras proximal 20 s'étend dans la direction de l'axe central A-A' au-delà de cette extrémité distale 43 du manchon proximal 38.

Comme précédemment, l'outil de largage 14 du dispositif comporte la tige interne, munie de la tête 92 de maintien d'une extrémité de l'implant 12, et le tuteur 93 monté coulissant coaxialement sur la tige.

L'outil de largage 14 comporte par ailleurs une gaine intérieure 94, montée coulissante par rapport au tuteur 93, une gaine intermédiaire 95 montée coulissante autour de la gaine intérieure 94, et une gaine extérieure 96 montée coulissante autour de la gaine intermédiaire 95.

Le tuteur 93 et les gaines 94, 95, 96 sont déplaçables à coulissement, indépendamment l'une de l'autre, et par rapport à la tige.

Des organes de verrouillage (non représentés) sont prévus entre la tige et le tuteur 93, entre le tuteur 93 et les gaines 94, 95, 96 pour éviter le coulissement spontané du tuteur 93, des gaines externes 96, intermédiaire 95 et internes 94. Ceci permet de procéder par étapes successives aux retraits de la gaine externe 96, de la gaine intermédiaire 95, de la gaine interne 94, et à la libération de l'extrémité proximale.

La tête 92 délimite le logement 100 de réception de l'extrémité proximale 44 de l'implant 12, dans lequel l'extrémité proximale 44 est maintenue comprimée radialement. Cette extrémité 44 est par ailleurs fixée axialement par rapport au tuteur 93.

Le tuteur 93 et la gaine interne 94 délimitent entre eux l'espace annulaire interne 102, destiné à recevoir le manchon distal 40 et les bras distaux 18. Ainsi, le manchon distal 40 est maintenu en configuration rétractée par cette gaine interne 94.

La gaine interne 94 et la gaine intermédiaire 95 délimitent entre elles ensemble un espace annulaire intermédiaire 103, destiné à recevoir le manchon proximal 38. Ainsi, le manchon proximal 38 est maintenu en configuration rétractée par cette gaine intermédiaire 95.

La gaine externe 96 et la gaine intermédiaire 95 délimitent entre elles l'espace annulaire exterieur 104, destiné à recevoir les bras proximaux 20. Ainsi, chaque bras proximal 20 est plaqué contre la gaine externe 96.

Lorsque l'implant 12 doit être positionné, notamment en remplacement d'une valve native, il est introduit entre les feuillets 110 de la valve native autour du siège 112 de la valve. Cette introduction peut être effectuée, à l'aide du dispositif de la Figure 16, en passant à travers le ventricule gauche, comme cela sera décrit ci-dessous en référence aux Figures 17 à 22.

Comme cela est représenté sur la Figure 17, la tête 92 et la partie aval de l'outil 14 est introduite dans la cavité atriale, au-delà de l'anneau mitral, de sorte que les bras distaux 18 soient disposés dans la cavité atriale au-delà de l'anneau mitral.

La gaine externe 96 est rétractée axialement à l'écart de la tête 92 par rapport à la gaine intermédiaire 95, la gaine interne 94 et par rapport au tuteur 93, pour découvrir les bras distaux 18, qui se déploient alors. L'outil 14 est alors déplacé vers le ventricule gauche pour plaquer les bras distaux 18 contre la face atriale des feuillets 110.

Comme cela est représenté sur la Figure 18, la gaine externe 96 est encore rétractée axialement pour découvrir les bras proximaux 20. Ceux-ci, qui étaient maintenus plaqués contre cette gaine externe 96, se déploient alors, notamment par élasticité. L'écartement des bras proximaux 20 est suffisant pour que les feuillets 110 se trouvent en regard de l'espace de réception 39 défini entre ces bras proximaux 20.

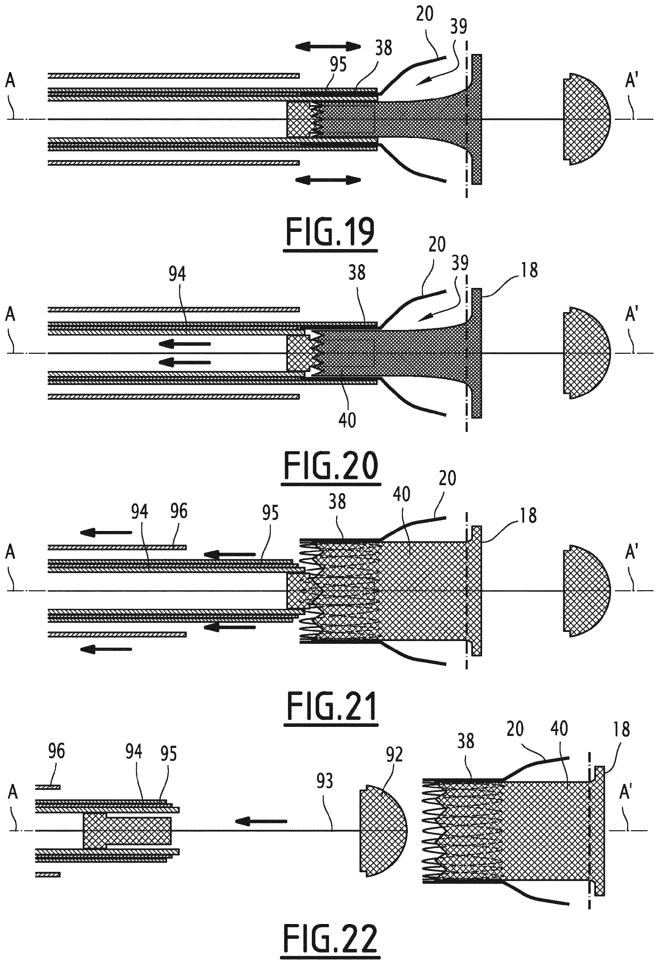

Comme cela est représenté sur la Figure 19, il suffit alors de déplacer le manchon proximal 38 et la gaine intermédiaire 95 qui l'entoure, en direction des feuillets 110. Ces feuillets 110 sont ainsi insérés dans l'espace de réception 39 défini entre les bras proximaux 20. Du fait que les bras proximaux 20 s'étendent au-delà de l'extrémité distale 43 du manchon proximal 38, ce manchon proximal 38 ne gêne pas l'insertion des feuillets 110 dans cet espace de réception 39.

Une fois le manchon proximal 38 en place, la gaine interne 94 est rétractée de manière à libérer le manchon distal 40. Ce manchon distal 40 étant disposé à l'intérieur du manchon proximal 38, il est maintenu en configuration contractée par ce manchon proximal 38, lui-même maintenu en configuration contractée par la gaine intermédiaire 95.

En se déployant à l'intérieur du manchon proximal 38, le manchon distal 40 se lie à ce manchon proximal 38.

On notera que, dans cette position, les feuillets 110 de la valve native sont intercalés entre le manchon distal 40 et les bras proximaux 20.

Une fois les manchons proximal 38 et distal 40 ainsi liés, on rétracte la gaine intermédiaire 95, de manière à libérer le manchon proximal 38, comme cela est représenté sur la Figure 21.

Le manchon proximal 38 se déploie radialement alors jusqu'à sa configuration déployée, ainsi que le manchon distal 40 à l'intérieur de ce manchon proximal 38 déployé.

Ceci étant fait, la fixation axiale entre le tuteur 93 et l'extrémité proximale 44 est libérée. Le tuteur 93, la tête 92 et la tige sont alors retirés hors du patient à travers le conduit interne, comme cela est représenté sur la figure 22.

On notera que, dans cette configuration déployée, le manchon distal 40 s'étend longitudinalement, en partie à l'intérieur du manchon proximal 38, coaxialement à ce manchon proximal 38, en partie à l'intérieur de l'espace 39 de réception de tissu, de sorte que le tissu est reçu entre les bras proximaux 20 et le manchon distal 40, et en partie au-delà de l'espace 39 de réception de tissu.

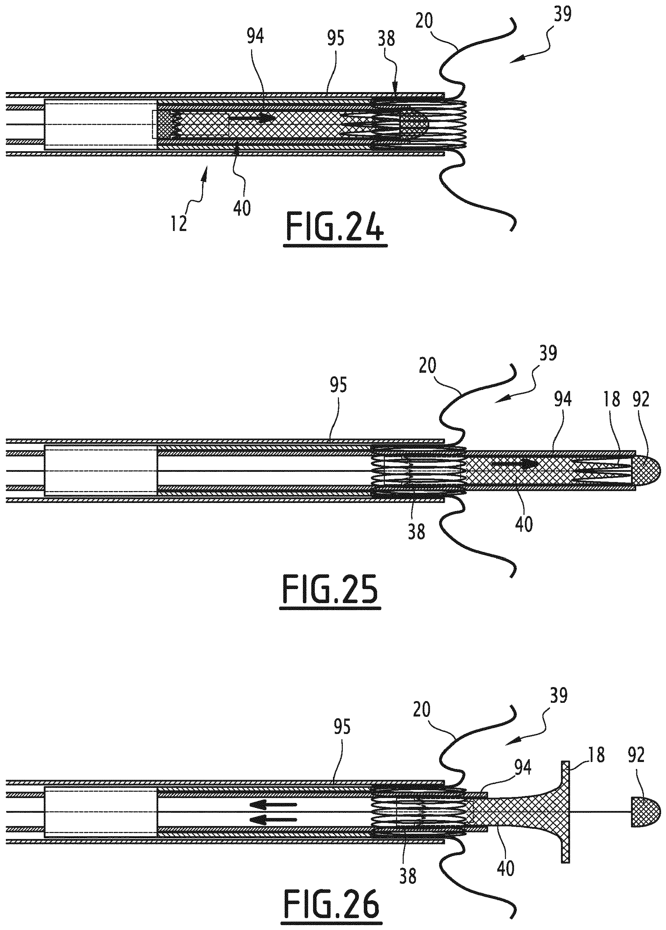

L'implant 12 peut également être introduit selon un autre procédé, à l'aide d'un dispositif similaire à celui de la Figure 16, en passant à travers le ventricule gauche, comme cela sera décrit ci-dessous en référence aux Figures 24 à 26.

Sur ces figures, l'implant 12 est légèrement différent de celui décrit précédemment, en ce que la forme de ses bras proximaux 20 est différente. Toutefois, le procédé d'introduction décrit précédemment, ou celui qui va maintenant être décrit, peuvent être indifféremment utilisés quelque soit la forme des bras proximaux 20 de l'implant 12.

Par ailleurs, l'outil de largage 14 ne comporte pas nécessaire de gaine externe, mais de préférence uniquement une gaine interne 94, maintenant le manchon distal 40 en position contractée, et une autre gaine, qui sera appelée gaine intermédiaire 95 par soucis de cohérence avec le mode de réalisation précédent, maintenant le manchon proximal 38 en position contractée.

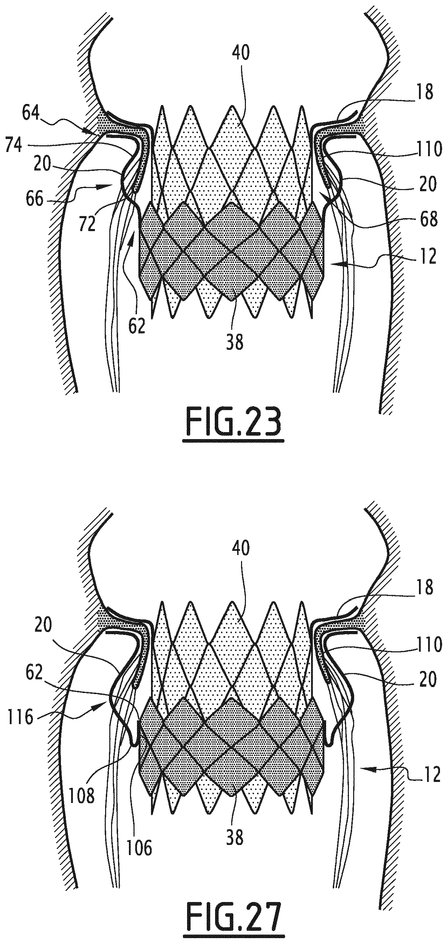

A l'exception de cette forme des bras proximaux 20, qui sera décrite ultérieurement plus en détail en référence à la figure 23, et de l'absence de gaine externe, l'outil de largage 14 et le reste de l'implant 12 sont similaires à ceux décrits en référence aux figures 16 à 22.

Conformément à cet autre procédé d'introduction, le manchon proximal 38 est déployé avant le manchon distal 40. Ainsi, comme cela est représenté sur la figure 24, la gaine intermédiaire 95 est rétractée axialement de manière à libérer les bras proximaux 20, qui se déploient alors, notamment par élasticité. L'écartement des bras proximaux 20 définit ainsi l'espace de réception 39, qui est alors entièrement libre.

Cet espace de réception 39 est disposé en regard des feuillets 110, afin qu'ils soient insérés dans cet espace de réception 39 par déplacement de l'outil de largage 14 en direction de ces feuillets 110.

Ce n'est qu'ensuite que la tête 92, et la gaine interne 94 renfermant le manchon distal 40, sont déplacés axialement jusqu'à la cavité atriale, au-delà de l'anneau mitral, de sorte que les bras distaux 18 soient disposés dans la cavité atriale au-delà de l'anneau mitral, comme cela est représenté sur la figure 25.

Ensuite, comme cela est représenté sur la figure 26, la gaine interne 94 est rétractée axialement à l'écart de la tête 92 pour découvrir les bras distaux 18, qui se déploient alors. L'outil 14 est alors déplacé vers le ventricule gauche pour plaquer les bras distaux 18 contre la face atriale des feuillets 110.

La position du manchon proximal 38 est également ajustée, puis la gaine interne 94 est entièrement rétractée. Le manchon distal 40 étant disposé à l'intérieur du manchon proximal 38, il est maintenu en configuration contractée par ce manchon proximal 38, lui-même maintenu en configuration contractée par la gaine intermédiaire 95. On se retrouve alors dans une configuration similaire à celle de la figure 20.

En se déployant à l'intérieur du manchon proximal 38, le manchon distal 40 se lie à ce manchon proximal 38.

On notera que, dans cette position, les feuillets 110 de la valve native sont intercalés entre le manchon distal 40 et les bras proximaux 20.

Une fois les manchons proximal 38 et distal 40 ainsi liés, on rétracte la gaine intermédiaire 95, de manière à libérer le manchon proximal 38, de manière similaire à la figure 21.

Le manchon proximal 38 se déploie radialement alors jusqu'à sa configuration déployée, ainsi que le manchon distal 40 à l'intérieur de ce manchon proximal 38 déployé.

Ceci étant fait, la fixation axiale entre le tuteur 93 et l'extrémité proximale est libérée. Le tuteur 93, la tête 92 et la tige sont alors retirés hors du patient à travers le conduit interne, de manière similaire à la figure 22.

L'implant 12 en position déployée est représenté sur la figure 23. Comme précédemment, dans cette configuration déployée, le manchon distal 40 s'étend longitudinalement, en partie à l'intérieur du manchon proximal 38, coaxialement à ce manchon proximal 38, en partie à l'intérieur de l'espace 39 de réception de tissu, de sorte que le tissu est reçu entre les bras proximaux 20 et le manchon distal 40, et en partie au-delà de l'espace 39 de réception de tissu.

On notera que la position relative du manchon proximal 38 par rapport au manchon distal 40 est choisie au cours de l'installation de l'implant 12, en fonction de la configuration du passage de circulation de sang dans lequel est installé cet implant 12. Ainsi, conformément à ce mode de réalisation, les manchons proximal 38 et distal 40 sont susceptibles de coulisser l'un par rapport à l'autre.

Conformément à cette variante de réalisation, chaque bras proximal 20 présente une forme bombée, de convexité dirigée radialement à l'écart de l'axe A-A'. En particulier, la région intermédiaire 66 de chaque bras proximal 20 comprend au moins un tronçon proximal 72 divergeant radialement à l'écart de l'extrémité liée 62 et au moins un tronçon distal 74 convergeant radialement vers l'extrémité libre 64. Cette forme bombée permet de définir une loge 68 longitudinale de réception d'un feuillet 110 de valves, comme cela a été décrit précédemment.

Comme indiqué précédemment, la forme des bras proximaux 20 n'influe pas sur le procédé d'introduction de l'implant, qui peut ainsi être similaire à celui décrit en référence aux figures 17 à 22, à celui décrit en référence aux figures 24 à 26, ou à tout autre procédé d'introduction envisageable.

Des exemples de formes de bras proximaux 20 vont maintenant être décrits, en référence aux figures 27 à 30. Sur ces figures, les éléments analogues à ceux des autres figures sont désignés par des références identiques.

On a représenté sur la figure 27 un implant 12 selon une autre variante de réalisation des bras proximaux 20.

Conformément à cette variante de réalisation, chaque bras proximal 20 comprend :
- une partie proximale 106, partant axialement depuis l'extrémité liée 62 en direction de l'extrémité proximale 41 du manchon proximal 38,
- une partie de bifurcation 108, reliant le tronçon proximal 106 à une partie distale 116 du bras proximal 20,
- la partie distale 116, partant axialement depuis la partie de bifurcation 108 en direction de l'extrémité distale du manchon proximal 38.

La partie distale 116 peut également présenter une forme bombée 66 telle que celle décrite précédemment en référence à la figure 23.

La partie proximale 106 et la partie de bifurcation 108 définissent ensemble une loge plus profonde pour les feuillets 110. Bien évidemment, la longueur de la partie proximale 106 peut être choisie plus ou moins importante, en fonction de la profondeur souhaitée pour la loge 68.

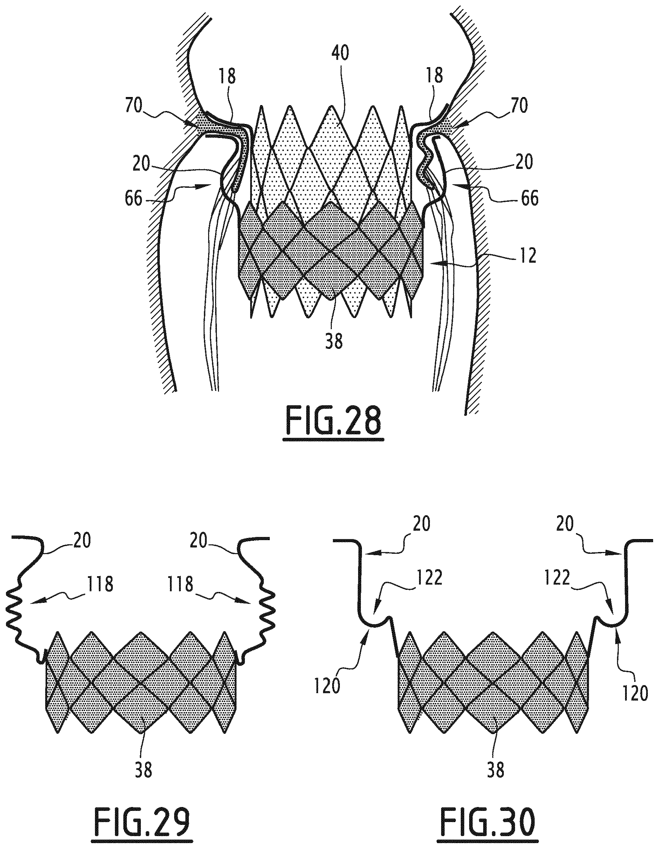

On a représenté sur la figure 28 un implant 12 selon une autre variante de réalisation.

Conformément à cette variante, au moins un bras proximal 20 présente une région distale 70 faisant saillie radialement à l'écart de l'axe central A-A' par rapport à la région intermédiaire 66.

Plus particulièrement, au moins un premier bras proximal 20 présente une première région distale 70 appliquée en regard d'un premier bras distal 18, et un deuxième bras proximal 20 présente une deuxième région distale 70 appliquée en regard d'un deuxième bras distal 18, l'étendue radiale de la première région distale 70 étant supérieure à l'étendue radiale de la deuxième région distale 70.

En termes généraux, l'étendue radiale de la région distale 70 de chaque bras proximal 20 peut être choisie plus ou moins grande, notamment en fonction de la forme prédéterminée du passage de circulation de sang destiné à recevoir l'implant.

On a représenté sur la figure 29 un manchon proximal 38 d'un implant 12 selon une autre variante de réalisation.

Conformément à cette variante, chaque bras proximal 20 comporte une partie intermédiaire 118 déformable élastiquement selon une direction longitudinale du bras proximal 20. A cet effet, la partie intermédiaire 118 présente généralement la forme d'un ressort.

Grâce à sa partie intermédiaire 118 longitudinalement déformable élastiquement, la longueur de chaque bras proximal 20 peut varier, et s'adapter en fonction du positionnement du manchon proximal 38 par rapport au manchon distal 40, notamment afin d'assurer un pincement optimal des feuillets 110 entre les bras proximaux 20 et les bras distaux 18. En d'autres termes, en l'absence de feuillets 110, il est possible d'assurer un contact entre l'extrémité libre 64 d'au moins un bras proximal 20 et un bras distal 18, dans la configuration déployée et en l'absence de sollicitation extérieure.

On a représenté sur la figure 30 un manchon proximal 38 d'un implant 12 selon une autre variante de réalisation.

Conformément à cette variante, chaque bras proximal 20 comporte une partie intermédiaire 120 pliée de manière à former un retour axial délimitant une cavité 122. Cette cavité 122 est destinée à former une loge pour les feuillets 110.

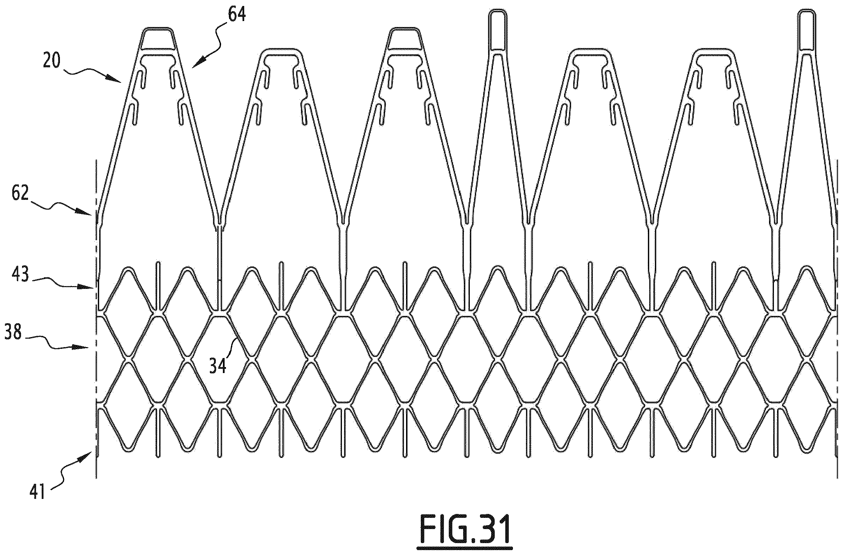

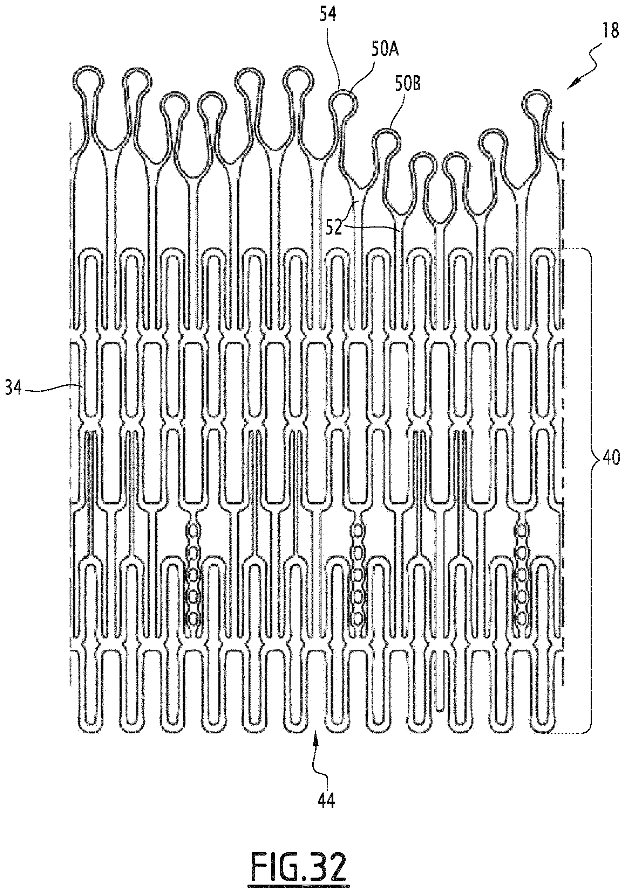

On a représenté sur les figures 31 et 32 un implant 12 selon une autre variante de réalisation. En particulier, le manchon proximal 38 de l'implant 12 est représenté sur la figure 31, et le manchon distal 40 sur la figure 32.

Le manchon proximal 38 est formé d'éléments filiformes 34 disposés en grillage, formant par exemple des mailles en losange.

Comme cela est notamment représenté sur la Figure 31, le manchon proximal 38, s'étend longitudinalement entre une extrémité proximale 41 et une extrémité distale 43 de manchon. Chaque bras proximal 20 s'étend entre une extrémité 62 liée à l'extrémité distale 43 du manchon proximal 38, et une extrémité libre 64. Ainsi, chaque bras proximal 20 s'étend dans la direction de l'axe central A-A' au-delà de cette extrémité distale 43 du manchon proximal 38.

Conformément à cette variante de réalisation, au moins un bras proximal 20 présente une longueur supérieure à celle d'au moins un autre bras proximal. En variante ou en combinaison avec ladite longueur supérieure, au moins un bras proximal 20 présente une extrémité liée 62 de largeur inférieure à celle de l'extrémité liée 62 d'au moins un autre bras proximal 20. Ainsi, les formes des bras proximaux 20 peut être adaptée à la configuration prédéterminée du passage de circulation de sang destiné à recevoir l'implant 12.

Par ailleurs, le manchon distal 40 est formé d'éléments filiformes 34 ondulés. On notera que ce manchon distal 40 présente une longueur axiale supérieure à celle du manchon distal qui a été décrit en référence à la figure 4.

Le manchon proximal 38 est destiné à être rapporté sur ce manchon distal 40, et il est susceptible de coulisser le long de ce manchon distal 40 jusqu'à être disposé à un position optimale par rapport à ce manchon distal 40, en fonction de la configuration du passage de circulation de sang destiné à recevoir l'implant 12.

Dans l'exemple représenté sur la Figure 32, les bras distaux 18 s'étendent continûment sur toute la périphérie du manchon distal 40 autour de l'axe A-A' en étant adjacents les uns aux autres.

Chaque bras distal 18 est formé par une boucle 50A, 50B présentant deux tronçons 52 intérieurs communs avec un bras distal 18 adjacent, et un tronçon extérieur propre 54 replié en boucle.

Comme précédemment, les bras distaux 18 sont mobiles entre une position axiale, représentée sur la figure 32, lorsque l'implant 12 occupe sa configuration contractée, et une position transversale, lorsque l'implant 12 occupe sa configuration déployée.

Dans la position transversale, au repos, en l'absence de sollicitation extérieure, chaque bras distal 18 s'étend perpendiculairement à l'axe A-A'.

La forme et la longueur des bras distaux 18 peut varier d'un bras à l'autre, comme cela est représenté sur la figure 32, notamment afin de s'adapter à la configuration prédéterminée du passage de circulation de sang destiné à recevoir l'implant 12.

On notera que l'invention n'est pas limitée aux modes de réalisation précédemment décrits, mais pourrait présenter diverses variantes sans sortir du cadre des revendications.

En particulier, on peut prévoir toutes sortes de formes adaptées pour les bras proximaux 20 et/ou les bras distaux 18.

Par exemple, en variante des bras proximaux 20 en forme de V inversé décrits précédemment, dont l'extrémité liée 62 présente une largeur supérieure à celle de l'extrémité libre 64, on peut prévoir au moins un bras proximal de forme évasée, dont l'extrémité liée 62 présente une largeur inférieure à celle de l'extrémité libre 64.

On peut également prévoir en variante au moins un bras proximal 20 de forme arrondie, en demi cercle ou en demi ovale.

## Revendications

1. Implant (12) destiné à être placé dans un passage de circulation du sang, et à être fixé sur un tissu, l'implant (12) étant déployable entre une configuration contractée et une configuration déployée, l'implant (12) comprenant :
- une armature tubulaire (16) autour d'un axe central (A-A'), définissant un conduit interne (32) de circulation du sang, l'armature tubulaire (16) s'étendant entre une extrémité proximale (44) et une extrémité distale (46) ;
- une pluralité de bras distaux (18) s'étendant perpendiculairement à l'axe central (A-A') dans la configuration déployée pour s'appuyer sur une première face du tissu ;
- une pluralité de bras proximaux (20) présentant une extrémité (62) liée à l'armature et une extrémité libre (64) destinée à s'appuyer sur une deuxième face du tissu pour pincer le tissu ;
**caractérisé en ce que**
- l'implant comprend un premier ensemble d'un seul tenant comportant une première partie (38) de l'armature et les bras proximaux (20), et un deuxième ensemble d'un seul tenant comportant une deuxième partie de l'armature (40) et les bras distaux (18), le premier ensemble et le deuxième ensemble étant rapportés l'un sur l'autre ;
- la première partie (38) de l'armature présente la forme d'un manchon proximal, d'axe central (A-A'), s'étendant longitudinalement entre une extrémité proximale (41) et une extrémité distale (43) de manchon ;
- l'extrémité liée (62) de chaque bras proximal (20) est liée à l'extrémité distale (43) du manchon proximal (38), et l'extrémité libre (64) de chaque bras proximal (20) s'étend dans la direction de l'axe central (A-A') au-delà de cette extrémité distale (43) du manchon proximal (38).

2. Implant (12) selon la revendication 1, dans lequel, les bras proximaux (20) délimitant entre eux un espace (39) de réception du tissu, le manchon proximal (38) s'étend intégralement en dehors de cet espace de réception (39).

3. Implant (12) selon la revendication 1 ou 2, dans lequel les première (38) et seconde (40) parties de l'armature sont chacune formée par un treillis de fils entrelacés, délimitant par exemple des mailles polygonales.

4. Implant (12) selon l'une quelconque des revendications précédentes, dans lequel :
- les bras proximaux (20) définissent entre eux un espace (39) de réception du tissu,
- la seconde partie (40) de l'armature présente la forme d'un manchon distal, d'axe central (A-A'), ce manchon distal (40) s'étendant longitudinalement :
• en partie à l'intérieur du manchon proximal (38), coaxialement à ce manchon proximal (38),
• en partie à l'intérieur de l'espace (39) de réception de tissu, de sorte que le tissu est susceptible d'être reçu entre les bras proximaux (20) et le manchon distal (40), et
• en partie au-delà de l'espace (39) de réception de tissu.

5. Implant (12) selon l'une quelconque des revendications précédentes, dans lequel, dans la configuration déployée, en l'absence de sollicitation extérieure, l'extrémité libre (64) d'au moins un bras proximal (20) est disposée au contact d'un bras distal (18) et/ou de l'armature (16), le bras proximal (20) comportant au moins une région intermédiaire (66) s'étendant le long et à l'écart radialement de l'armature (16) pour définir une loge longitudinale (68) de réception du tissu.

6. Implant (12) selon la revendication 5, dans lequel la région intermédiaire (66) de chaque bras proximal (20) présente une forme bombée, de convexité dirigée radialement à l'écart de l'axe (A-A'), la région intermédiaire (66) comprenant au moins un tronçon proximal (72) divergeant radialement à l'écart de l'extrémité liée (62) et au moins un tronçon distal (74) convergeant radialement vers l'extrémité libre (64).

7. Implant (12) selon la revendication 5 ou 6, dans lequel au moins un bras proximal (20) définit, au niveau de son extrémité libre (64), une région distale (70) faisant saillie radialement à l'écart de l'axe central (A-A') par rapport à la région intermédiaire (66), la région distale (70) étant appliquée sous un bras distal (18), la rigidité en flexion de la région intermédiaire (66) étant de préférence supérieure à la rigidité en flexion de la région distale (70).

8. Implant (12) selon l'une quelconque des revendications précédentes, dans lequel chaque bras proximal (20) comporte deux branches (78) convergeant distalement l'une vers l'autre pour présenter sensiblement une forme de V retourné en configuration déployée.

9. Implant (12) selon l'une quelconque des revendications précédentes, dans lequel chaque bras distal (18) forme une boucle (50A, 50B) faisant saillie transversalement par rapport à l'axe central (A-A').

10. Implant (12) selon la revendication 9, dans lequel les bras distaux (18) sont adjacents les uns aux autres pour former une collerette (60) transversale perpendiculaire à l'axe (A-A') dans la configuration déployée, la collerette (60) étant avantageusement couverte d'une jupe en tissu propre à guider le sang à travers l'armature.

11. Implant (12) selon l'une quelconque des revendications précédentes, dans lequel l'étendue radiale d'un premier bras distal (18) dans un premier secteur angulaire (S1) autour de l'axe (A-A') est supérieure à l'étendue radiale d'un deuxième bras distal (18) situé dans un deuxième secteur angulaire (S2), avantageusement supérieure à 50% de l'étendue radiale d'un deuxième bras distal (18).

12. Implant (12) selon l'une quelconque des revendications précédentes, dans lequel au moins un premier bras proximal (20) présente une première région distale (70) appliquée en regard d'un premier bras distal (18), un deuxième bras proximal (20) présentant une deuxième région distale (70) appliquée en regard d'un deuxième bras distal (18), l'étendue radiale de la première région distale (70) étant supérieure à l'étendue radiale de la deuxième région distale (70).

13. Implant (12) selon l'une quelconque des revendications précédentes, dans lequel le manchon proximal (38) présente, lorsqu'il est séparé du manchon distal (40), et en l'absence de sollicitation extérieure, un diamètre inférieur à celui du manchon distal (40) en l'absence de sollicitation extérieure.

14. Dispositif (10 ; 210) de traitement d'un passage de circulation du sang, **caractérisé en ce qu'**il comporte :
- un implant (12) selon l'une quelconque des revendications précédentes ;
- un outil (14) de largage de l'implant (12), l'implant (12) étant monté dans sa configuration contractée dans l'outil de largage (14).

15. Dispositif (10) selon la revendication 14, comportant une tête (92) de blocage délimitant au moins un logement (100) de réception de l'armature (16), un tuteur (93) engagé dans l'armature (16), une gaine interne (94) de retenue de chaque bras distal (18) dans une configuration axiale disposée autour de l'armature (16) et une gaine externe (96) de plaquage de chaque bras proximal (20) contre la gaine externe (96), la gaine interne (92) étant disposée autour de l'armature (16) et de chaque bras distal (18) pour maintenir le bras distal dans une configuration axiale.

16. Dispositif (210) selon la revendication 14, comportant une tête (92) de blocage délimitant au moins un logement (100) de réception de l'armature (16) et de retenue de chaque bras distal (18) dans une configuration axiale, un tuteur (93) engagé à travers l'armature (16), une gaine externe (96) de placage de chaque bras proximal (20) contre l'armature (16), l'armature (16) et les bras distaux (18) étant sensiblement immobiles par rapport au tuteur (93) lors du déplacement de la gaine externe (96), le dispositif (210) comportant en outre une paroi intermédiaire (220) faisant saillie à partir de la tête (92) dans la gaine externe (96) pour maintenir en position les bras proximaux (20) et les séparer des bras distaux (18).

17. Dispositif selon la revendication 14, comportant :
- une tête (92) de blocage délimitant au moins un logement (100) de réception de l'armature (16),
- un tuteur (93) engagé dans l'armature (16), comprenant une extrémité distale fixée à la tête de blocage (92),
- une gaine interne (94) de retenue de la deuxième partie de l'armature (40) en configuration contractée, la gaine interne (92) étant disposée autour de la deuxième partie de l'armature (40),
- une gaine externe (96) de plaquage de chaque bras proximal (20) contre la gaine externe (96), la gaine externe (96) étant disposée autour des bras proximaux (20) ;
- une gaine intermédiaire (95) de retenue de la première partie de l'armature (38) en configuration contractée, la gaine intermédiaire étant disposée autour de la première partie de l'armature (38), cette première partie (38) étant disposée entre la gaine interne (94) et la gaine intermédiaire (95).

18. Dispositif selon l'une quelconque des revendications précédentes, dans lequel chaque bras proximal (20) comporte, entre son extrémité liée (62) et son extrémité libre (64), au moins une région intermédiaire (66) s'étendant le long et à l'écart radialement de l'armature (16) pour définir une loge (68) longitudinale de réception d'un feuillet de valves.

## Patentansprüche

1. Implantat (12), das dazu bestimmt ist, in einer Blutzirkulationspassage platziert zu werden und an einem Gewebe fixiert zu werden, wobei das Implantat (12) entfaltbar ist zwischen einer Kontraktions-Konfiguration und einer Entfaltungs-Position, wobei das Implantat (12) aufweist:
- eine rohrförmige Armierung (16) um eine zentrale Achse (A-A') herum, die eine innere Blutzirkulations-Leitung (32) definiert, wobei die rohrförmige Armierung (16) sich zwischen einem proximalen Ende (44) und einem distalen Ende (46) erstreckt,
- ein Mehrzahl von distalen Armen (18), die sich in der Entfaltungs-Konfiguration senkrecht zu der zentralen Achse (A-A') erstrecken, um sich an einer ersten Fläche des Gewebes abzustützen,
- eine Mehrzahl von proximalen Armen (20), die ein Ende (62), das mit der Armierung verbunden ist, und ein freies Ende (64) haben, das dazu bestimmt, sich auf einer zweiten Fläche des Gewebes abzustützen, um das Gewebe zu einzuklemmen,
**dadurch gekennzeichnet, dass**
- das Implantat aufweist eine erste Einrichtung aus einem einzigen Festhalter, der einen ersten Abschnitt (38) der Armierung und die proximalen Arme (20) aufweist, und eine zweite Einrichtung aus einem einzigen Festhalter, der einen zweiten Abschnitt der Armierung (40) und die distalen Arme (18) aufweist, wobei die erste Einrichtung und die zweite Einrichtung miteinander verbunden sind,
- wobei der erste Abschnitt (38) der Armierung die Form einer proximalen Muffe, mit zentraler Achse (A-A'), hat, die sich längs zwischen einem proximalen Ende (41) und einem distalen Ende (43) der Muffe erstreckt,
- wobei das verbundene Ende (62) jedes proximalen Arms (20) mit dem distalen Ende (43) der proximalen Muffe (38) verbunden ist, und wobei das freie Ende (64) jedes proximalen Arms (20) sich in der Richtung der zentralen Achse (A-A') über dieses distale Ende (43) der proximalen Muffe (38) hinauserstreckt.

2. Implantat (12) gemäß Anspruch 1, wobei die proximalen Arme (20) zwischen sich einen Raum (39) zur Aufnahme des Gewebes begrenzen und die proximale Muffe (38) sich integral außerhalb dieses Aufnahme-Raums (39) erstreckt.

3. Implantat (12) gemäß Anspruch 1 oder 2, wobei der erste (38) und der zweite (40) Abschnitt der Armierung jeweils von einem Gitter verflochtener Drähte gebildet ist, das zum Beispiel polygonale Maschen begrenzt.

4. Implantat (12) gemäß irgendeinem der vorhergehenden Ansprüche, wobei:
- die proximalen Arme (20) zwischen sich einen Raum (39) zur Aufnahme des Gewebes definieren,
- wobei der zweite Abschnitt (40) der Armierung die Form einer distalen Muffe, mit zentraler Achse (A-A'), hat, wobei sich die distale Muffe (40) längs erstreckt:
- teilweise im Inneren der proximalen Muffe (38) koaxial zu dieser proximalen Muffe (38),
- teilweise im Inneren des Raums (39) zur Aufnahme von Gewebe, so dass das Gewebe imstande ist, zwischen den proximalen Armen (20) und der distalen Muffe (40) aufgenommen zu werden, und
- teilweise über den Raum (39) zur Aufnahme von Gewebe hinaus.

5. Implantat (12) gemäß irgendeinem der vorhergehenden Ansprüche, wobei in der Entfaltungskonfiguration, in Abwesenheit einer äußeren Beanspruchung, das freie Ende (64) wenigstens eines proximalen Arms (20) im Kontakt mit einem distalen Arm (18) und/oder mit der Armierung (16) angeordnet ist, wobei der proximale Arm (20) wenigstens einen Zwischenbereich (66) aufweist, der sich entlang und im Radialabstand von der Armierung (16) erstreckt zum Definieren einer Längsaufnahme (68) zur Aufnahme des Gewebes.

6. Implantat (12) gemäß Anspruch 5, wobei der Zwischenbereich (66) jedes proximalen Arms (20) eine bauchige Form hat, mit einer Konvexität, die radial von der Achse (A-A') weg weist, wobei der Zwischenbereich (66) aufweist wenigstens einen proximalen Abschnitt (72), der von dem freien Ende (62) weggehend radial divergiert, und wenigstens einen distalen Abschnitt (74), der zu dem freien Ende (64) hin radial konvergiert.

7. Implantat (12) gemäß Anspruch 5 oder 6, wobei wenigstens ein proximaler Arm (20), auf Niveau dessen freien Endes (64), einen distalen Bereich (70) definiert, der bezüglich des Zwischenbereichs (66) radial von der zentralen Achse (A-A') weg vorsteht, wobei der distale Bereich (70) unter einem distalen Arm (18) angewendet ist, wobei die Biegesteifheit des Zwischenbereichs (66) bevorzugt größer ist als die Biegesteifheit des distalen Bereichs (70).

8. Implantat (12) gemäß irgendeinem der vorhergehenden Ansprüche, wobei jeder proximale Arm (20) aufweist zwei Zweige (78), die distal zueinander hin konvergieren, um in der Entfaltungs-Konfiguration im Wesentlichen eine Form eines umgedrehten V zu haben.

9. Implantat (12) gemäß irgendeinem der vorhergehenden Ansprüche, wobei jeder distale Arm (18) eine Schleife (50A, 50B) bildet, die bezüglich der zentralen Achse (A-A') quer vorsteht.

10. Implantat (12) gemäß Anspruch 9, wobei die distalen Arme (18) benachbart zueinander sind, um in der Entfaltungs-Konfiguration einen Quer-Kragen (60) senkrecht zu der Achse (A-A') zu bilden, wobei der Kragen (60) vorteilhafterweise einen Rock aus Gewebe abdeckt, imstande das Blut durch die Armierung hindurch zu leiten.

11. Implantat (12) gemäß irgendeinem der vorhergehenden Ansprüche, wobei die radiale Erstreckung eines ersten distalen Arms (18) in einem ersten Winkelbereich (S1) um die Achse (A-A') größer ist als die radiale Erstreckung eines zweiten distalen Arms (18), der in einem zweiten Winkelbereich (S2) angeordnet ist, vorteilhafterweise um 50% größer als die radiale Erstreckung eines zweiten distalen Arms (18).

12. Implantat (12) gemäß irgendeinem der vorhergehenden Ansprüche, wobei wenigstens ein erster proximaler Arm (20) einen ersten distalen Bereich (70) hat, der gegenüber einem ersten distalen Arm (18) angewandt ist, wobei ein zweiter proximaler Arm (20) einen zweiten distalen Bereich (70) hat, der gegenüber einem zweiten distalen Arm (18) angewandt ist, wobei die radiale Erstreckung des ersten distalen Bereichs (70) größer ist als die radiale Erstreckung des zweiten distalen Bereichs (70).

13. Implantat (12) gemäß irgendeinem der vorhergehenden Ansprüche, wobei die proximale Muffe (38), wenn sie von der distalen Muffe (40) separiert ist und in Abwesenheit von einer äußeren Beanspruchung, einen Durchmesser hat, der kleiner ist als jener der distalen Muffe (40) in Abwesenheit einer äußeren Beanspruchung.

14. Vorrichtung (10; 210) zur Behandlung einer Blutzirkulationspassage, **dadurch gekennzeichnet, dass** sie aufweist:
- ein Implantat (12) gemäß irgendeinem der vorhergehenden Ansprüche,
- ein Werkzeug (14) zum Abwerfen des Implantats (12), wobei das Implantat (12) in dessen Kontraktions-Konfiguration in dem Abwurf-Werkzeug (14) montiert ist.

15. Vorrichtung (10) gemäß Anspruch 14, aufweisend einen Kopf (92) zur Verriegelung, welcher wenigstens eine Unterbringung (100) zur Aufnahme der Armierung (16) begrenzt, eine Stütze (93), die mit der Armierung (16) im Eingriff ist, eine innere Hülle (94) zum Rückhalten jedes distalen Arms (18) in einer Axial-Konfiguration, angeordnet um die Armierung (16) herum, und eine äußere Hülle (96) zum Flachmachen jedes proximalen Arms (20) gegen die äußere Hülse (96), wobei die innere Hülle (92) um die Armierung (16) und jeden distalen Arm (18) herum angeordnet ist zum Halten des distalen Arms in einer Axial-Konfiguration.

16. Vorrichtung (210) gemäß Anspruch 14, aufweisend einen Kopf (92) zur Verriegelung, der wenigstens eine Unterbringung (100) zur Aufnahme der Armierung (16) und zum Rückhalten jedes distalen Arms (18) in einer Axial-Konfiguration begrenzt, eine Stütze (93), die durch die Armierung (16) hindurch im Eingriff ist, eine äußere Hülle (96) zum Flachmachen jedes proximalen Arms (20) gegen die Armierung (16), wobei die Armierung (16) und die distalen Arme (18) im Wesentlichen immobilisiert sind bezüglich der Stütze (93) während der Verlagerung der äußeren Hülle (96), wobei die Vorrichtung (210) ferner aufweist eine Zwischenwand (220), die von dem Kopf (92) ausgehend vorsteht in die äußere Hülle (96), um die proximalen Arme (20) in Position zu halten und diese von den distalen Armen zu separieren.

17. Vorrichtung gemäß Anspruch 14, aufweisend:
- einen Kopf (92) zur Verriegelung, der wenigstens eine Unterbringung (100) zur Aufnahme der Armierung (16) begrenzt,
- eine Stütze (93), die in der Armierung (16) in Eingriff ist, aufweisend ein distales Ende, das an dem Verriegelungs-Kopf (92) befestigt ist,
- eine innere Hülle (94) zum Rückhalten des zweiten Abschnitts der Armierung (40) in Kontraktions-Konfiguration, wobei die innere Hülle (92) um den zweiten Abschnitt der Armierung (40) herum angeordnet ist,
- eine äußere Hülle (96) zum Flachmachen jedes proximalen Arms (20) gegen die äußere Hülle (96), wobei die äußere Hülle (96) um die proximalen Arme (20) herum angeordnet sind,
- eine Zwischenhülle (95) zum Rückhalten des ersten Abschnitts der Armierung (38) in Kontraktions-Konfiguration, wobei die Zwischenhülle um den ersten Abschnitt der Armierung (38) herum angeordnet ist, wobei dieser erste Abschnitt (38) zwischen der inneren Hülle (94) und der Zwischenhülle (95) angeordnet ist.

18. Vorrichtung gemäß irgendeinem der vorhergehenden Ansprüche, wobei jeder proximale Arm (20) zwischen seinem verbundenen Ende (62) und seinem freien Ende (64) wenigstens einen Zwischenbereich (66) aufweist, der sich entlang und in radialem Abstand von der Armierung (16) erstreckt zum Definieren einer Längs-Unterbringung (68) zur Aufnahme eines Ventilblatts.

## Claims

1. An implant (12) intended to be placed in a blood flow passage and to be fixed on a tissue, the implant (12) being deployable between a contracted configuration and a deployed configuration, the implant (12) comprising:
- a tubular armature (16) around a central axis (A-A'), defining an inner blood flow conduit (32), the tubular armature (16) extending between a proximal end (44) and a distal end (46);
- a plurality of distal arms (18) extending perpendicular to the central axis (A-A') in the deployed configuration to bear on a first face of the tissue;
- a plurality of proximal arms (20) having an end (62) connected to the armature and a free end (64) intended to bear on a second face of the tissue to pinch the tissue; **characterized in that**
- the implant comprises a first assembly in one piece including a first part (38) of the armature and the proximal arms (20), and a second assembly in a single piece including a second part of the armature (40) and the distal arms (18), the first assembly and the second assembly being attached on one another;
- the first part (38) of the armature is in the form of a proximal sleeve, with central axis (A-A'), extending longitudinally between a first end (41) and a distal end (43) of the sleeve;
- the connected end (62) of each proximal arm (20) is connected to the distal end (43) of the proximal sleeve (38), and the free end (64) of each proximal arm (20) extends toward the central axis (A-A') beyond this distal end (43) of the proximal sleeve (38).

2. The implant (12) according to claim 1, wherein, the proximal arms (20) delimiting a space (39) between them for receiving the tissue, the proximal sleeve (38) extends completely outside this receiving space (39).

3. The implant (12) according to claim 1 or 2, wherein the first (38) and second (40) parts of the armature are each formed by a web of interlaced yarns, for example delimiting polygonal meshes.

4. The implant (12) according to any one of the preceding claims, wherein:
- the proximal arms (20) define a space (39) between them for receiving the tissue,
- the second part (40) of the armature is in the form of a distal sleeve, with central axis (A-A'), this distal sleeve (40) extending longitudinally:
• partly inside the proximal sleeve (38), coaxially to this proximal sleeve (38),
• partly inside the tissue receiving space (39), such that the tissue is able to be received between the proximal arms (20) and the distal sleeve (40), and
• partly beyond the tissue receiving space (39).

5. The implant (12) according to any one of the preceding claims, wherein, in the deployed configuration, without outside stress, the free end (64) of at least one proximal arm (20) is arranged in contact with a distal arm (18) and/or the armature (16), the proximal arm (20) including at least one intermediate region (66) extending along and radially separated from the armature (16) to define a longitudinal housing (68) for receiving the tissue.

6. The implant (12) according to claim 5, wherein the intermediate region (66) of each proximal arm (20) has a curved shape, with a convex side oriented radially away from the axis (A-A'), the intermediate region (66) comprising at least one proximal segment (72) diverging radially away from the connected end (62) and at least one distal segment (74) converging radially toward the free end (64).

7. The implant (12) according to claim 5 or 6, wherein at least one proximal arm (20) defines, at its free end (64), a distal region (70) protruding radially away from the central axis (A-A') relative to the intermediate region (66), the distal region (70) being pressed below a distal arm (18), the flexural rigidity of the intermediate region (66) preferably being greater than the flexural rigidity of the distal region (70).

8. The implant (12) according to any one of the preceding claims, wherein each proximal arm (20) includes two branches (78) converging distally toward one another so as substantially to have an inverted V shape in the deployed configuration.

9. The implant (12) according to any one of the preceding claims, wherein each distal arm (18) forms a loop (50A, 50B) protruding transversely relative to the central axis (A-A').

10. The implant (12) according to claim 9, wherein the distal arms (18) are adjacent to one another to form a transverse flange (60) perpendicular to the axis (A-A') in the deployed configuration, the flange (60) advantageously being covered by a fabric skirt able to guide the blood through the armature.

11. The implant (12) according to any one of the preceding claims, wherein the radial expanse of a first distal arm (18) in a first angular sector (S1) around the axis (A-A') is greater than the radial expanse of a second distal arm (18) situated in a second angular sector (S2), advantageously greater than 50% of the radial expanse of a second distal arm (18).

12. The implant (12) according to any one of the preceding claims, wherein at least a first proximal arm (20) has a first distal region (70) applied across from a first distal arm (18), a second proximal region (20) having a second distal region (70) applied across from a second distal arm (18), the radial expanse of the first distal region (70) being greater than the radial expanse of the second distal region (70).

13. The implant (12) according to any one of the preceding claims, wherein the proximal sleeve (38) has, when it is separated from the distal sleeve (40), and without outside stress, a diameter smaller than that of the distal sleeve (40) without outside stress.

14. A device (10, 210) for treating a blood flow passage, **characterized in that** it includes:
- an implant (12) according to any one of the preceding claims;
- a tool (14) for releasing the implant (12), the implant (12) being mounted in its contracted configuration in the release tool (14).

15. The device (10) according to claim 14, including a blocking head (92) delimiting at least one housing (100) for receiving the armature (16), a stay (93) engaged in the armature (16), an inner sheath (94) for retaining each distal arm (18) in an axial configuration arranged around the armature (16) and an outer sheath (96) for pressing each proximal arm (20) against the outer sheath (96), the inner sheath (92) being arranged around the armature (16) and each distal arm (18) to keep the distal arm in an axial configuration.

16. The device (210) according to claim 14, including a blocking head (92) delimiting at least one housing (100) for receiving the armature (16) and retaining each distal arm (18) in an axial configuration, a stay (93) engaged through the armature (16), an outer sheath (96) for pressing each proximal arm (20) against the armature (16), the armature (16) and the distal arms (18) being substantially immobile relative to the stay (93) during the movement of the outer sheath (96), the device (210) further including an intermediate wall (220) protruding from the head (92) and the outer sheath (96) to keep the proximal arms (20) in position and separate them from the distal arms (18).

17. The device according to claim 14, including:
- a blocking head (92) delimiting at least one housing (100) for receiving the armature (16),
- a stay (93) engaged in the armature (16), comprising a distal end fastened to the blocking head (92),
- an inner sheath (94) for keeping the second part of the armature (40) in the contracted configuration, the inner sheath (92) being arranged around the second part of the armature (40),
- an outer sheath (96) for pressing each proximal arm (20) against the outer sheath (96), the outer sheath (96) being arranged around proximal arms (20);
- an intermediate sheath (95) for keeping the first part of the armature (38) in the contracted configuration, the intermediate sheath being arranged around the first part of the armature (38), this first part (38) being arranged between the inner sheath (94) and the intermediate sheath (95).

18. The device according to any one of the preceding claims, wherein each proximal arm (20) includes, between its connected end (62) and its free end (64), at least one intermediate region (66) extending along and radially separated from the armature (16) to define a longitudinal housing (68) for receiving a valve leaflet.
